# EUROPEAN PATENT APPLICATION

(11) **EP 4 345 113 A1**
(43) Date of publication of application: **03.04.2024**
(21) Application number: 23198278.6
(22) Date of filing: 19.09.2023
(51) Int. Cl.: C07K 16/32, G01N 33/574, G01N 33/577

(54) **ANTI-HER2 ANTIBODY, A NUCLEIC ACID MOLECULE ENCODING THE VARIABLE REGION OF SAID ANTIBODY, A METHOD OF DETECTING HER2 IN A BIOLOGICAL SAMPLE, AN IMMUNO-ENZYMATIC ELISA ASSAY AND USE OF SAID ANTI-HER2 ANTIBODY OR FRAGMENTS THEREFOF**

(30) Priority: 30.09.2022 EP 22199237; 30.09.2022 EP 22199245
(71) Applicant: SDS Optic Spolka Akcyjna, 20-612 Lublin (PL)
(72) Inventor: Staniszewska, Magdalena, 21 030 Motycz (PL); Staniszewski, Marcin, 21 030 Motycz (PL); Lipinski, Tomasz, 54 066 Wroclaw (PL); Topolska Wos, Agnieszka, 20 583 Lublin (PL); Wos, Marcin, 20 583 Lublin (PL); Kuzmicz Miroslaw, Ewelina, 20 388 Abramowice Prywatne (PL); Glen, Justyna, 20 719 Lublin (PL); Antos, Aleksandra, 21 100 Lubartow (PL)
(74) Representative: Kondrat, Mariusz

(57) **Abstract**

Invention relates to an anti-HER2 antibody, characterised in that it comprises: a) a heavy chain variable region comprising CDR1, CDR2 and CDR3, in which CDR1 is an amino acid sequence of GYSFTGYN (SEQ ID No. 1) or GYSFTDYN (SEQ ID No. 2), CDR2 is an amino acid sequence of IDPNYGT (SEQ ID No. 3) or IDHYNGNT (SEQ ID No. 4), CDR3 is an amino acid sequence of ARRKVNYEAWFAF (SEQ ID No. 5) or ARHYDYGAMDY (SEQ ID No. 6); b) a light chain variable region comprising CDR1, CDR2 and CDR3 in which CDR1 is an amino acid sequence of ESVDSYGNSF (SEQ ID No. 7) or QNVGTN (SEQ ID No. 8), CDR2 is an amino acid sequence of RAS (SEQ ID No. 9) or SAS (SEQ ID No. 10), CDR3 constitutes an amino acid sequence of QQSNEDPFT (SEQ ID No. 11) or QHYYIYPLT (SEQ ID No. 12). Invention further relates to a nucleic acid molecule encoding the variable region of an antibody according to invention or a fragment thereof. Invention further relates to a method of detecting HER2 in a biological sample, comprising contacting the sample with an HER2-binding protein and detecting a complex, wherein the detection of said complex indicates HER2 expression in the sample, characterised in that HER2-binding protein is the antibody according to invention. Invention further relates to an immuno-enzymatic ELISA assay for the detection of HER2 antigen, characterised in that at least one antibody according to invention is used for the detection of antigen. Invention further relates to the use of antibody according to invention for producing antibody-drug conjugates and to the use of SEQ ID No. 17-28 nucleotide sequences encoding variable regions of the antibodies according to invention for the CAR-T production.

## Description

The invention relates to a novel anti-HER2 antibody and a nucleic acid molecule encoding a variable region of this antibody. The invention also relates to a method for detecting HER2 in a biological sample, to an immuno-enzymatic ELISA assay, and to the use of said anti-HER2 antibody or fragments thereof.

The *human epidermal growth factor* receptor 2 (HER2) is a member of the epidermal growth factor receptor (EGFR) family and is involved, among others, in cell proliferation processes. A rapid growth of HER2 levels has been confirmed in approximately 20% of breast cancers and 20% of gastric cancers. In both of these cases, it is associated with a worse prognosis of patients, hence it is an attractive target as a prognostic factor. Structurally, an approximately 185 kDa receptor consists of an extracellular domain (ECD), an α-helical transmembrane domain and an intracellular domain with tyrosine kinase activity. The HER2 receptor can dimerise with other epidermal growth factor (EGF) receptors, and HER2/HER3 dimer is the most active form that stimulates tumorigenesis. The cytoplasmic tyrosine kinase domain is phosphorylated during receptor activation and transmits a signal in the form of a cascade of activation of subsequent target proteins into the cell. The signalling cascade thus activated stimulates cell division while inhibiting apoptosis, which can lead to tumour growth. Extracellular domain of ECD is released during receptor activation and can be detected afterwards in the extracellular space and peripheral blood. Such indirect HER2 receptor expression measurements by determining the concentration of the free extracellular domain in blood serum can be used, among others, to assess the prognosis of breast cancer patients or for the choice of therapeutic method, depending on the HER2 marker status. It is assumed that physiological serum HER2 levels in healthy women should not exceed 15 ng/ml *(*Schwartz MK, Smith C, Schwartz DC, Dnistrian A, Neiman I. Monitoring Therapy by Serum HER-2/Neu, Internation T Biol Markers. 2000;15(4):324-329. doi: 10.11771172460080001500409*;* Zhang Z, Li C, Fan H, Xiang Q, Xu L, Liu β, Zhou S, Xie Q, Chen S, Mu G, Cui Y. Prognostic value of baseline serum HER2 extracellular domain level with a cut-off value of 15 ng/mL in patients with breast cancer: a systematic review and meta-analysis. Breast Cancer Res Treat. 2018;172(3):513-521. doi: 10.1007/s10549-018-4942-4*).* Elevated HER2 levels noted in gastric cancer may be subject to detection in peritoneal fluid and postoperative washings, providing an important source of material to assess patient prognosis.

Therefore, there is a need to develop new, more effective anti-HER2 antibodies and efficient ways to detect HER2 expression in biological samples for use in the diagnosis of HER-positive tumours.

The use of anti-HER2 antibodies in cancer treatment per se is known in the art. For example, application WO2020221791 A1 relates to a monoclonal anti-HER2 antibody or a functional fragment or derivative thereof in combination with a second HER2 inhibitor, for use in the prevention or treatment of disease associated with HER2 overexpression, amplification and/or overactivation. The disclosed antibodies are directed against an epitope between amino acids 342-652 of human HER2 for use in combination with a second HER2 inhibitor. More specifically, the discussed application relates to methods and uses of MAB270 or antibodies having the same CDRs as MAB270 in combination with trastuzumab or pertuzumab in HER2 positive cancer.

Application WO2021170071 A1 discloses a multispecific antibody that binds to CD137 and HER2, comprising a first antibody construct that binds to CD137 and a second antibody construct that binds to HER2.

Application US5821337 A discloses recombinant humanised antibodies, including a recombinant humanised version of a murine HER2 antibody, along with methods for their manufacture and use. Consensus immunoglobulin sequences and structural models are also provided. Whereby, a disclosed method of producing a humanised antibody comprising the amino acid sequence of an imported non-human antibody and a human antibody comprises: obtaining the amino acid sequences of at least a portion of the variable domain of the imported antibody and the consensus variable domain; identifying the amino acid sequences of complementarity determining region (CDR) in the imported and human variable domain sequences; substituting the imported amino acid sequence of the CDR with the corresponding amino acid sequence of the human CDR; aligning the amino acid sequences of framework region (FR) of the imported antibody and the corresponding FR of the consensus antibody. Identification of FR residues of the imported antibody in matched FR sequences that are non-homologous with the corresponding consensus antibody residues; determination of whether the imported non-homologous amino acid residue can reasonably be expected to have at least one of the following effects: non-covalently direct antigen binding; interacting with CDR; or participating in the VL-VH interface; and for any amino acid residue of a non-homologous imported antibody that can reasonably be expected to have at least one of these effects, replacing that residue with the corresponding amino acid residue in the FR sequence of the consensus antibody.

Application US2020048362 discloses a method of treatment of cancer that overexpresses ErbB2 in a mammal comprising administering an anti- ErbB2 antibody-maytansinoid conjugate, wherein the anti-ErbB2 antibody is a growth inhibitory antibody that binds to ErbB2 and inhibits the growth of cancer cells overexpressing ErbB2. Advantageously, the anti-ErbB2 antibody binds epitope 4D5 to which the antibody 4D5 (ATCC CRL 10463) binds or binds essentially the same epitope as the 4D5 antibody.

Application US2002090662 discloses a method of accurately determining the amount of antibody in a fluid sample in the presence of an interfering substance capable of binding said antibody, comprising: providing a solid surface dual-coated with a first antibody recognizing said free antibody and a second antibody recognizing said interfering substance upon binding to said antibody; contacting said dual-coated surface with said fluid sample, whereby said first antibody binds said free antibody and said second antibody binds said interfering substance bound to said antibody; and determining the total amount of said free antibody and said antibody bound to said interfering substance. Advantageously, said antibody is anti-HER2 antibody and said interfering substance is an extracellular domain (ECD) of the HER2 oncoprotein. Furthermore, in preferred embodiments, said anti-HER2 antibody is a murine anti-HER2 monoclonal antibody (e.g., 4D5 or is a humanised version of a recombinant 4D5 antibody).

The invention aims to provide a new specific anti-HER2 antibody and a new efficient method of HER2 detection in a biological sample, and to provide an efficient method of measurement of the extracellular domain of ECD in a solution, including blood serum.

The subject of invention is an anti-HER2 antibody, characterised in that it comprises:
a) a heavy chain variable region comprising CDR1, CDR2 and CDR3, wherein CDR1 is an amino acid sequence GYSFTGYN (SEQ ID No. 1) or GYSFTDYN (SEQ ID No. 2);
   CDR2 is an amino acid sequence of IDPNYGT (SEQ ID No. 3) or IDHYNGNT (SEQ ID No. 4);
   CDR3 is an amino acid sequence of ARRKVNYEAWFAF (SEQ ID No. 5) or ARHYDYGAMDY (SEQ ID No. 6);
b) A light chain variable region comprising CDR1, CDR2 and CDR3 in which
   CDR1 is an amino acid sequence of ESVDSYGNSF (SEQ ID No. 7) or QNVGTN (SEQ ID No. 8);
   CDR2 is an amino acid sequence of RAS (SEQ ID No. 9) or SAS (SEQ ID No. 10);
   CDR3 is an amino acid sequence QQSNEDPFT (SEQ ID No. 11) or QHYYIYPLT (SEQ ID No. 12);

Advantageously, the anti-HER2 antibody according to the invention comprises:
a) a heavy chain with a heavy chain variable region comprising or consisting of an amino acid sequence of SEQ ID No. 13 with 0, 1, 2, 3, 4 or 5 amino acid insertions, deletions, substitutions or additions, or an amino acid sequence of SEQ ID No. 14 with 0, 1, 2, 3, 4 or 5 amino acid insertions, deletions or additions;
b) a light chain with a light chain variable region comprising or consisting of an amino acid sequence of SEQ ID 15 with 0, 1, 2, 3, 4 or 5 amino acid insertions, deletions, substitutions or additions, or an amino acid sequence of SEQ ID 16 with 0, 1, 2, 3, 4 or 5 amino acid insertions, deletions or additions.

Advantageously, the heavy chain variable region of the anti-HER2 antibody according to the invention consists of amino acid sequence of SEQ ID No. 13 and its light chain variable region consists of amino acid sequence of SEQ ID No. 15.

Advantageously, the heavy chain variable region of the anti-HER2 antibody according to the invention consists of amino acid sequence of SEQ ID No. 14 and its light chain variable region consists of amino acid sequence of SEQ ID No. 16.

Another subject of invention is a nucleic acid molecule encoding the variable region of an antibody according to the invention or a fragment thereof.

Another subject of invention is a method for detecting HER2 in a biological sample, comprising contacting the sample with a HER2-binding protein and detecting a complex, wherein the detection of said complex indicates HER2 expression in the sample, characterised in that HER2-binding protein is an antibody according to the invention.

Advantageously, in the method according to the invention, contacting of the sample with the HER2-binding protein and the detection of the complex is carried out using an immunoenzymatic ELISA assay, wherein a first anti-HER2 antibody comprises a heavy chain variable region comprising CDR1 with the amino acid sequence of SEQ ID No. 1, CDR2 with amino acid sequence of SEQ ID No. 3, CDR3 with amino acid sequence of SEQ ID No. 5, and comprises a light chain variable region comprising CDR1 with amino acid sequence of SEQ ID No. 7, CDR2 with amino acid sequence of SEQ ID No. 9 and CDR3 with amino acid sequence of SEQ ID No. 11, while a second anti-HER2 antibody is a biotin-bound antibody that comprises a heavy chain variable region comprising CDR1 with amino acid sequence of SEQ ID No. 2, CDR2 with amino acid sequence of SEQ ID No. 4 and CDR3 with amino acid sequence of SEQ ID No. 6, and comprises a light chain variable region comprising CDR1 with amino acid sequence of SEQ ID No. 8, CDR2 with amino acid sequence of SEQ ID No. 10 and CDR3 with amino acid sequence of SEQ ID No. 12.

Advantageously, in the method according to the invention, contacting of the sample with the HER2-binding protein and the detection of the complex is carried out using an immunoenzymatic ELISA assay, wherein the first anti-HER2 antibody comprises a heavy chain variable region comprising or consisting of amino acid sequence of SEQ ID No. 13, and comprises a light chain variable region comprising or consisting of amino acid sequence of SEQ ID No. 15, and the second anti-HER2 antibody is a biotin-bound antibody that comprises a heavy chain variable region comprising or consisting of amino acid sequence of SEQ ID No. 14 and comprises a light chain variable region comprising or consisting of amino acid sequence of SEQ ID No. 16.

Advantageously, in the method according to the invention, contacting of the sample with the HER2-binding protein and the detection of the complex is carried out using an optoelectronic biosensor with anti-HER2 antibodies according to the invention and/or fragments thereof immobilised on the surface of said sensor.

Advantageously, biological sample is blood serum, peritoneal fluid, cell lysate, tissue homogenate, cell filtrate, supernatant or recombinant antigen suspended in solution.

A further subject of the invention is an immuno-enzymatic ELISA assay for the detection of HER2 antigen, characterised in that at least one antibody according to the invention is used for the detection of the antigen. Advantageously, two antibodies according to the invention are used for detecting the HER2 antigen, the of which the fist antibody is an immobilized antibody that comprises a heavy chain variable region comprising CDR1 with amino acid sequence of SEQ ID No. 1, CDR2 with amino acid sequence of SEQ ID No. 3, CDR3 with amino acid sequence of SEQ ID No. 5, and comprises a light chain variable region comprising CDR1 with amino acid sequence of SEQ ID No. 7, CDR2 with amino acid sequence of SEQ ID No. 9 and CDR3 with amino acid sequence of SEQ ID No. 11, while the second anti-HER2 antibody is a biotin-bound antibody that comprises a heavy chain variable region comprising CDR1 with amino acid sequence of SEQ ID No. 2, CDR2 with amino acid sequence of SEQ ID No. 4 and CDR3 with amino acid sequence of SEQ ID No. 6, and comprises a light chain variable region comprising CDR1 with amino acid sequence of SEQ ID No. 8, CDR2 with amino acid sequence of SEQ ID No. 10 and CDR3 with amino acid sequence of SEQ ID No. 12.

Another subject of the invention is an use of an antibody according to the invention for producing antibody-drug conjugates.

Another subject of the invention is an use of the nucleotide sequences of SEQ ID No. 17-28 encoding the variable regions of the antibodies according to the invention for CAR-T production.

The invention provides the following advantages:
- Antigen binding strength, which remains the main parameter characterising the antibodies according to the invention, and antigen binding specificity allows detection of sole, true antigen, without cross-reactivity and detection of so-called off-targets;
- The strength of antigen binding as determined by the ED50 value, i.e., half of the effective dose, is at or between 0.0922 nM and 0.2347 nM;
- The test sample can be serum, cell lysate, peritoneal fluid or tissue homogenate;
- The invention provides a sensitive immunoenzymatic ELISA assay with a relatively short reading time;
- Increased test sensitivity - ELISA assay sensitivity threshold lowered to 0.5 ng/ml;
- The immuno-enzymatic assay according to the invention allows the quantification of HER2 within the working range of the standard curve of: 15.6-100 ng/ml. Determination of higher concentrations of HER2 is possible by performing dilutions of the test samples. The use of dilutions of the test samples allowed the determination of HER2 antigen concentrations as high as 150 µg/ml (data obtained for murine tumours);
- The method according to the invention using an immuno-enzymatic assay makes it possible to stop the procedure with maintaining of stability of the immobilised components (i.e., the antibodies according to the invention), which significantly shortens the procedure for analysing samples and makes it possible to distribute the coated plates as an intermediate between laboratories in order to standardise analyses;
- Therapeutic use of humanised anti-HER2 antibodies according to the invention in antibody-drug conjugates (ADCs) is possible;
- Therapeutic use of the nucleotide sequences encoding the variable regions of the antibodies according to the invention for the production of CAR-T is possible;
- The antibodies according to the invention and/or their F(ab')2 fragments can be used to produce an optoelectronic biosensor.

The term "antibodies", as used herein, is meant to be interpreted as proteins with the ability to specifically attach to an antigen that are produced by stimulated B cells in the course of an immune response of the humoral type. This is made possible by their Y-like structure. A complete IgG class antibody consists of two long polypeptide chains referred to as heavy chains and two shorter light chains. These chains are linked to each other by disulphide bonds, which break down under reducing conditions. In each antibody, there is a constant part, which remains identical among antibodies of the same class, and a variable part, which allows specific recognition of antigens. As a result of digestion, it is possible to separate the antigen-binding fragment of the antibody, so-called F(ab')2 (fragment, antigen-binding), which constitutes the arms of the antibody and lacking fragment crystallizable region (Fc region).

Depending on the method of obtaining and possible selection, the population of antibodies recognising the same antigen can be homo- or heterogeneous. Homogeneous antibodies that recognise the same epitope of antigen are referred to as monoclonal and are obtained by detailed selection. Polyclonal antibodies, on the other hand, are a heterogeneous mixture recognising different epitopes of the same antigen and are not subjected to detailed selection in the process of obtaining them, and their specificity is usually lower.

In a first aspect, the invention relates to an anti-HER2 antibody comprising:
a) a heavy chain variable region comprising CDR1, CDR2 and CDR3, in which
   CDR1 is an amino acid sequence of GYSFTGYN (SEQ ID No. 1) or GYSFTDYN (SEQ ID No. 2),
   CDR2 is an amino acid sequence of IDPNYGT (SEQ ID No. 3) or IDHYNGNT (SEQ ID No. 4),
   CDR3 is an amino acid sequence of ARRKVNYEAWFAF (SEQ ID No. 5) or ARHYDYGAMDY (SEQ ID No. 6);
b) a light chain variable region comprising CDR1, CDR2 and CDR3 in which
   CDR1 is an amino acid sequence of ESVDSYGNSF (SEQ ID No. 7) or QNVGTN (SEQ ID No. 8),
   CDR2 is an amino acid sequence of RAS (SEQ ID No. 9) or SAS (SEQ ID No. 10),
   CDR3 is an amino acid sequence QQSNEDPFT (SEQ ID No. 11) or QHYYIYPLT (SEQ ID No. 12).

Whereby, CDR stands for complementarity-determining region and FR stands for framework region.

The antibodies of the invention are monoclonal antibodies with high specificity towards HER2.

Whereby, the terms "in an embodiment", "in one embodiment" or "in one of embodiments", as used herein, is meant to be interpreted as in one or more embodiments. Furthermore, the features present in the various embodiments may be combined with each other. The descriptions of the embodiments of the invention in the present application are given by way of example and are not intended to limit the scope of the invention. The described embodiments include various features, not all of which are required in all embodiments of the invention. Some embodiments use only some of the features or possible combinations of features. The described variants of the invention and embodiments of the invention comprising different combinations of the features mentioned in the described embodiments will come to the mind of experts in the field. The scope of the invention is limited only by the claims.

In one embodiment, the heavy chain variable region of the antibody according to the invention comprises CDR1 having the amino acid sequence of GYSFTGYN (SEQ ID No. 1), CDR2 having the amino acid sequence of IDPNYGT (SEQ ID No. 3) and CDR3 having the amino acid sequence of ARRKVNYEAWFAF (SEQ ID No. 5). Wherein, the light chain variable region comprises CDR1 with the amino acid sequence of ESVDSYGNSF (SEQ ID No. 7), CDR2 with the amino acid sequence of RAS (SEQ ID No. 9) and CDR3 with the amino acid sequence of QQSNEDPFT (SEQ ID No. 11).

In another embodiment, the heavy chain variable region of the antibody according to the invention comprises CDR1 having the amino acid sequence of GYSFTDYN (SEQ ID No. 2), CDR2 having the amino acid sequence of IDHYNGNT (SEQ ID No. 4) and CDR3 having the amino acid sequence of ARHYDYGAMDY (SEQ ID No. 6). Wherein, the light chain variable region comprises CDR1 with the amino acid sequence of QNVGTN (SEQ ID No. 8), CDR2 with the amino acid sequence of SAS (SEQ ID No. 10) and CDR3 with the amino acid sequence of QHYYIYPLT (SEQ ID No. 12).

In one embodiment, the anti-HER2 antibody according to the invention comprises a heavy chain with the heavy chain variable region comprising the amino acid sequence of SEQ ID No. 13 with either 0, 1, 2, 3, 4 or 5 insertions, deletions of substitutions or amino acid additions, and the light chain variable region comprises the amino acid sequence of SEQ ID 15 with either 0, 1, 2, 3, 4 or 5 insertions, deletions of substitutions or amino acid additions.

In one embodiment, the strength of antigen binding in the form of the determination of the ED50 value, i.e., half of the effective dose, is at 0.0922 nM.

In another embodiment, the anti-HER2 antibody according to the invention comprises a heavy chain with the heavy chain variable region consisting of the amino acid sequence of SEQ ID No. 13 with either 0, 1, 2, 3, 4 or 5 insertions, deletions of substitutions or amino acid additions, and the light chain variable region consisting of the amino acid sequence of SEQ ID 15 with either 0, 1, 2, 3, 4 or 5 insertions, deletions of substitutions or amino acid additions.

In another embodiment, the anti-HER2 antibody according to the invention comprises a heavy chain with the heavy chain variable region comprising the amino acid sequence of SEQ ID No. 14 with either 0, 1, 2, 3, 4 or 5 insertions, deletions of substitutions or amino acid additions, and the light chain variable region comprising the amino acid sequence of SEQ ID 16 with either 0, 1, 2, 3, 4 or 5 insertions, deletions of substitutions or amino acid additions.

In one embodiment, the strength of antigen binding as determined by the ED50 value, i.e., half of the effective dose, is at 0.2347 nM.

In another embodiment, the anti-HER2 antibody according to the invention comprises a heavy chain with the heavy chain variable region consisting of the amino acid sequence of SEQ ID No. 14 with either 0, 1, 2, 3, 4 or 5 insertions, deletions of substitutions or amino acid additions, and the light chain variable region consisting of the amino acid sequence of SEQ ID 16 with either 0, 1, 2, 3, 4 or 5 insertions, deletions of substitutions or amino acid additions.

In another embodiment, the heavy chain variable region of the antibody according to the invention is the amino acid sequence of SEQ ID No. 13 and its light chain variable region is the amino acid sequence of SEQ ID No. 15.

In another embodiment, the heavy chain variable region of the antibody according to the invention is the amino acid sequence of SEQ ID No. 14 and its light chain variable region is the amino acid sequence of SEQ ID No. 16.

In another embodiment, the heavy chain of the antibody according to the invention is an amino acid sequence of SEQ ID No. 29 or SEQ ID No. 29 with either 0, 1, 2, 3, 4, 5, 6, 7, 8, 9 or 10 amino acid insertions, deletions substitutions or additions.

In another embodiment, the heavy chain of the antibody according to the invention is an amino acid sequence of SEQ ID No. 30 or SEQ ID No. 30 with either 0, 1, 2, 3, 4, 5, 6, 7, 8, 9 or 10 amino acid insertions, deletions substitutions or additions.

In another embodiment, the light chain of the antibody according to the invention is an amino acid sequence of SEQ ID No. 31 or SEQ ID No. 31 with either 0, 1, 2, 3, 4, 5, 6, 7, 8, 9 or 10 amino acid insertions, deletions substitutions or additions.

In another embodiment, the light chain of the antibody according to the invention is an amino acid sequence of SEQ ID No. 32 or SEQ ID No. 32 with either 0, 1, 2, 3, 4, 5, 6, 7, 8, 9 or 10 amino acid insertions, deletions substitutions or additions.

In another embodiment, the heavy chain of the antibody according to the invention is an amino acid sequence of SEQ ID No. 29 or SEQ ID No. 29 with either 0, 1, 2, 3, 4, 5, 6, 7, 8, 9 or 10 amino acid insertions, deletions, substitutions or additions, while the light chain is an amino acid sequence of SEQ ID No. 31 or SEQ ID No. 31 with either 0, 1, 2, 3, 4, 5, 6, 7, 8, 9 or 10 amino acid insertions, deletions or additions.

In another embodiment, the heavy chain of the antibody according to the invention is an amino acid sequence of SEQ ID No. 30 or SEQ ID No. 30 with either 0, 1, 2, 3, 4, 5, 6, 7, 8, 9 or 10 amino acid insertions, deletions, substitutions or additions, while the light chain is an amino acid sequence of SEQ ID No. 32 or SEQ ID No. 32 with either 0, 1, 2, 3, 4, 5, 6, 7, 8, 9 or 10 amino acid insertions, deletions, substitutions or additions.

Monoclonal antibodies with high specificity towards HER2, which are the subject of the present invention, were obtained by immunizing mice with the corresponding antigen.

In one embodiment monoclonal antibodies with high specificity towards HER2 according to the invention were obtained by immunization of Balb/c mice with the corresponding antigen. A fusion of antibody-producing murine B cells with immortalised murine Sp2/O-Ag14 myeloma cells resulted in hybridoma cells. Under controlled laboratory conditions, these cells retain the immunological capabilities of short-lived B cells, while maintaining an unlimited capacity to divide. After appropriate selection, lines derived from single fusion cells produce antibodies identical in terms of recognizing of epitope.

In one embodiment, hybridoma cells were cultured at 37°C in an atmosphere of 5% CO₂ according to an established protocol. Briefly, I adherent and suspension hybridoma cells were initially cultured in rich DMEM (Dulbecco's Modified Eagle Medium) supplemented with 10% FBS (fetal bovine serum) until full confluence was achieved, i.e., a full coverage of the surface of the bottle or culture plate with a single layer. Cells in culture maintained a viability of 70-80%. The cells are then passaged and the medium was replaced with Panserin H4000 (PAN-Biotech, P04-714001) completely devoid of protein components. The highest efficiency of antibody production was achieved under concentrated culture conditions, so cultures are continued in a single bottle with regular replacement of the medium with fresh medium. The source of the antibodies is the culture medium, from which the antibodies are isolated by affinity chromatography. In one embodiment, the production yield from continuous culture of hybridoma cells ranges from 2.92 mg±1.6 to 3.98 mg±2.99 per litre of post culture medium.

In other embodiments, monoclonal antibodies according to the invention can also be produced by other techniques, including, but not limited to, the method of *in vivo* hybridoma cell culture in the peritoneal cavity of animals (providing a large number of specific antibodies from previously obtained hybridoma cells), recombinant DNA methods, phage library methods and methods using transgenic animals containing all or part of human immunoglobulin loci.

In a further aspect, the invention relates to a nucleic acid molecule encoding a variable region of an antibody according to the invention or a fragment thereof.

In one embodiment, the nucleic acid molecule encodes CDR1 of the variable heavy chain region of an antibody according to the invention having the sequence of SEQ ID No. 17. In another embodiment, the nucleic acid molecule encodes CDR1 of the variable heavy chain region of an antibody according to the invention having the sequence of SEQ ID No. 18.

In one embodiment, the nucleic acid molecule encodes CDR2 of the variable heavy chain region of an antibody according to the invention having the sequence of SEQ ID No. 19. In another embodiment, the nucleic acid molecule encodes CDR2 of the variable heavy chain region of an antibody according to the invention having the sequence of SEQ ID No. 20.

In one embodiment, the nucleic acid molecule encodes CDR3 of the variable heavy chain region of an antibody according to the invention having the sequence of SEQ ID No. 21. In another embodiment, the nucleic acid molecule encodes CDR3 of the variable heavy chain region of an antibody according to the invention having the sequence of SEQ ID No. 22.

In one embodiment, the nucleic acid molecule encodes CDR1 of the light chain variable region of an antibody according to the invention having the sequence of SEQ ID No. 23. In another embodiment, the nucleic acid molecule encodes CDR1 of the light chain variable region of an antibody according to the invention having the sequence of SEQ ID No. 24.

In one embodiment, the nucleic acid molecule encodes CDR2 of the light chain variable region of an antibody according to the invention having the sequence of SEQ ID No. 25. In another embodiment, the nucleic acid molecule encodes CDR2 of the light chain variable region of an antibody according to the invention having the sequence of SEQ ID No. 26.

In one embodiment, the nucleic acid molecule encodes CDR3 of the light chain variable region of an antibody according to the invention having the sequence of SEQ ID No. 27. In another embodiment, the nucleic acid molecule encodes CDR3 of the light chain variable region of an antibody according to the invention having the sequence of SEQ ID No. 28.

In one embodiment, the nucleic acid molecule encodes the variable region of the antibody heavy chain according to the invention having the sequence of SEQ ID No. 37. In another embodiment, the nucleic acid molecule encodes the variable region of the antibody heavy chain according to the invention having the sequence of SEQ ID No. 38.

In one embodiment, the nucleic acid molecule encodes the light chain variable region of an antibody according to the invention with sequence SEQ No. 39. In another embodiment, the nucleic acid molecule encodes the light chain variable region of an antibody according to the invention with sequence SEQ No. 40.

In a further aspect, the invention relates to a method for detecting HER2 in a biological sample which comprises contacting the sample with an antibody according to the invention and detecting the complex, the detection of the complex indicating the expression of HER2 in the sample.

In one embodiment, the biological sample is blood serum. In another embodiment, the biological sample is peritoneal fluid, cell lysate or tissue homogenate. In another embodiment, the biological sample is recombinant antigen suspended in solution (e.g., saline). In another embodiment, the biological sample is cell filtrate or cell culture supernatant.

In one embodiment, the method for detecting HER2 in a biological sample includes providing a solid surface (e.g. 96-well plate) coated with an antibody comprising a heavy chain variable region that comprises CDR1 having the amino acid sequence of SEQ ID No. 1, CDR2 having the amino acid sequence of SEQ ID No. 3, CDR3 having the amino acid sequence of SEQ ID No. 5, and the light chain variable region comprising CDR1 having the amino acid sequence of SEQ ID No. 7, CDR2 having the amino acid sequence of SEQ ID No. 9 and CDR3 having the amino acid sequence of SEQ ID No. 11. For this purpose, the specific antibody according to the invention is applied in carbonate buffer directly to the surface of the plate. This buffer allows the binding and stabilisation of the antibody to the surface of the plastic and the subsequent attachment of further assay components. The areas on the plate surface not bound by adhesins are blocked afterwards (e.g., with a solution of 5% milk in PBS). The plate is then fixed with trehalose to protect the bound antibody during drying and to stabilise this system during long-term storage. At this stage, the plate undergoes drying and can be stored at 5 °C for at least 4 months. Detection of recombinant HER2 antigen or native HER2 derived from human body fluid samples is carried out by applying the solution to the plate and incubating for one hour at ambient temperature. During this time, the HER2 antigen from the solution undergoes binding to the antibody according to the invention immobilised on the plate surface. In the process of washing the plate, the free unbound antigen is removed from the wells and another anti-HER2 antibody, this time as antibody in complex with biotin, is added to the conjugate of HER2 and the antibody according to the invention.

In one embodiment, the biotin-bound anti-HER2 antibody comprises a heavy chain variable region comprising CDR1 with the amino acid sequence of SEQ ID No. 2, CDR2 with the amino acid sequence of SEQ ID No. 4 and CDR3 with the amino acid sequence of SEQ ID No. 6, and the light chain variable region comprising CDR1 with the amino acid sequence of SEQ ID No. 8, CDR2 with the amino acid sequence of SEQ ID No. 10 and CDR3 with the amino acid sequence of SEQ ID No. 12.

In another embodiment, contacting the sample with the HER2-binding protein and detecting the complex is carried out using an immunoenzymatic ELISA assay, wherein the first anti-HER2 antibody comprising a heavy chain variable region that comprises or consists of the amino acid sequence of SEQ ID No. 13, and the light chain variable region that comprises or consists of the amino acid sequence of SEQ ID No. 15, and the second biotin-bound anti-HER2 antibody comprises a heavy chain variable region comprising or consisting of the amino acid sequence of SEQ ID No. 14 and comprises light chain variable region comprising or consisting the amino acid sequence of SEQ ID No. 16.

In one embodiment, the biological sample is blood serum, peritoneal fluid, cell lysate, tissue homogenate, cell filtrate, supernatant or recombinant antigen suspended in solution.

In preferred embodiment, the test sample is serum.

In another preferred embodiment, the method for detecting HER2 in a biological sample according to the invention comprising contacting the sample with a HER2-binding protein and detecting the complex is carried out using an optoelectronic biosensor with anti-HER2 antibodies according to the invention immobilised on its surface, which act as adhesins.

The biosensor technology with anti-HER2 antibodies and/or fragments of anti-HER2 antibodies according to the invention immobilised on their surface is sensitive enough to detect nanogram (including physiological) amounts of antigen, as well as elevated levels of HER2 as a result of pathological conditions, including in HER2-positive breast cancer.

The sensitivity of biosensors, combined with the possibility of application in the living organism represents an innovative technology that has been proposed as a modern tool for oncology diagnosis. In this case, the HER2-detecting biosensor can be used as a less invasive and less time-consuming solution than a biopsy, monitoring in real time, not only the levels of the breast cancer marker in the tumour's surroundings, but also drug delivery and efficacy. The biosensor, inserted in a biopsy needle into the tumour area, allows the result to be recorded within ~20 minutes.

Whereby, the biological sample for biosensor testing can also be blood serum, peritoneal fluid, cell lysate, tissue homogenate, cell filtrate, supernatant or recombinant antigen suspended in solution.

Whereby, in one embodiment, an antibody according to the invention comprising a heavy chain variable region that comprises CDR1 with the amino acid sequence of GYSFTGYN (SEQ ID No. 1), CDR2 with the amino acid sequence of IDPNYGT (SEQ ID No. 3) and CDR3 with the amino acid sequence of ARRKVNYEAWFAF (SEQ ID No. 5), was used. Wherein, the light chain variable region of used antibody comprises CDR1 with the amino acid sequence of ESVDSYGNSF (SEQ ID No. 7), CDR2 with the amino acid sequence of RAS (SEQ ID No. 9) and CDR3 with the amino acid sequence of QQSNEDPFT (SEQ ID No. 11).

In another embodiment, an antibody according to the invention is used, wherein the heavy chain variable region of the antibody according to the invention comprises CDR1 having the amino acid sequence of GYSFTDYN (SEQ ID No. 2), CDR2 having the amino acid sequence of IDHYNGNT (SEQ ID No. 4) and CDR3 having the amino acid sequence of ARHYDYGAMDY (SEQ ID No. 6). Wherein, the light chain variable region comprises CDR1 with the amino acid sequence of QNVGTN (SEQ ID No. 8), CDR2 with the amino acid sequence of SAS (SEQ ID No. 10) and CDR3 with the amino acid sequence of QHYYIYPLT (SEQ ID No. 12).

In one embodiment, the anti-HER2 antibody according to the invention comprises a heavy chain variable region comprising amino acid sequence of SEQ ID No. 13 with either 0, 1, 2, 3, 4 or 5 insertions, deletions of substitutions or amino acid additions, and the light chain variable region comprising amino acid sequence of SEQ ID 15 with either 0, 1, 2, 3, 4 or 5 insertions, deletions of substitutions or amino acid additions.

In another embodiment, the anti-HER2 antibody according to the invention comprises a heavy chain variable region consisting of the amino acid sequence of SEQ ID No. 13 with either 0, 1, 2, 3, 4 or 5 insertions, deletions of substitutions or amino acid additions, and the light chain variable region consisting of the amino acid sequence of SEQ ID 15 with either 0, 1, 2, 3, 4 or 5 insertions, deletions of substitutions or amino acid additions.

In another embodiment, the anti-HER2 antibody according to the invention comprises a heavy chain variable region comprising the amino acid sequence of SEQ ID No. 14 with either 0, 1, 2, 3, 4 or 5 insertions, deletions of substitutions or amino acid additions, and the light chain variable region comprises the amino acid sequence of SEQ ID 16 with either 0, 1, 2, 3, 4 or 5 insertions, deletions of substitutions or amino acid additions, is used.

In another embodiment, the anti-HER2 antibody according to the invention comprises a heavy chain variable region consisting of the amino acid sequence of SEQ ID No. 14 with either 0, 1, 2, 3, 4 or 5 insertions, deletions of substitutions or amino acid additions and the light chain variable region consisting of the amino acid sequence of SEQ ID 16 with either 0, 1, 2, 3, 4 or 5 insertions, deletions of substitutions or amino acid additions.

In another embodiment, the heavy chain variable region of the used antibody according to the invention consists of the amino acid sequence of SEQ ID No. 13 and its light chain variable region consists of the amino acid sequence of SEQ ID No. 15.

In another embodiment, the heavy chain variable region of the used antibody according to the invention consists the amino acid sequence of SEQ ID No. 14 and its light chain variable region consists of the amino acid sequence of SEQ ID No. 16.

In another embodiment, the heavy chain of the antibody according to the invention used consists of the amino acid sequence of SEQ ID No. 29 or SEQ ID No. 29 with either 0, 1, 2, 3, 4, 5, 6, 7, 8, 9 or 10 amino acid insertions, deletions, substitutions or additions.

In another embodiment, the heavy chain of the antibody according to the invention used consists of the amino acid sequence of SEQ ID No. 30 or SEQ ID No. 30 with either 0, 1, 2, 3, 4, 5, 6, 7, 8, 9 or 10 amino acid insertions, deletions, substitutions or additions.

In another embodiment, the light chain of the antibody according to the invention used consists of the amino acid sequence of SEQ ID No. 31 or SEQ ID No. 31 with either 0, 1, 2, 3, 4, 5, 6, 7, 8, 9 or 10 amino acid insertions, deletions, substitutions or additions.

In another embodiment, the light chain of the antibody according to the invention used consists of the amino acid sequence of SEQ ID No. 32 or SEQ ID No. 32 with 0, 1, 2, 3, 4, 5, 6, 7, 8, 9 or 10 amino acid insertions, deletions, substitutions or additions.

In another embodiment, the heavy chain of the antibody according to the invention used consists of the amino acid sequence of SEQ ID No. 29 or SEQ ID No. 29 with either 0, 1, 2, 3, 4, 5, 6, 7, 8, 9 or 10 insertions, substitution deletion or amino acid addition, while the light chain consists of the amino acid sequence of SEQ ID No. 31 or SEQ ID No. 31 with either 0, 1, 2, 3, 4, 5, 6, 7, 8, 9 or 10 insertions, substitution deletions or amino acid additions.

In another embodiment, the heavy chain of the antibody according to the invention used consists of the amino acid sequence of SEQ ID No. 30 or SEQ ID No. 30 with either 0, 1, 2, 3, 4, 5, 6, 7, 8, 9 or 10 insertions, substitution deletion or amino acid addition, while the light chain consists of the amino acid sequence of SEQ ID No. 32 or SEQ ID No. 32 with either 0, 1, 2, 3, 4, 5, 6, 7, 8, 9 or 10 insertions, substitution deletions or amino acid additions.

At the same time, anti-human HER2 antibodies immobilised on the surface of the biosensor, acting as adhesins, capture even small, nanogram quantities of HER2 antigen from the solution. Sensitive fibre-optic technology, on the other hand, makes it possible to record the signal, which is a spectral shift given in nanometres.

In a further aspect, the invention discloses an immuno-enzymatic ELISA assay for detecting the HER2 antigen, characterised in that at least one antibody according to the invention is used to detect the antigen.

In one embodiment, two antibodies according to the invention are used for the detection of the HER2 antigen, the first of which is an immobilized antibody whose heavy chain variable region comprises CDR1 comprising the amino acid sequence of SEQ ID No. 1, CDR2 comprising the amino acid sequence of SEQ ID No. 3, CDR3 comprising the amino acid sequence of SEQ ID No. 5, and the light chain variable region of said antibody comprises CDR1 comprising the amino acid sequence of SEQ ID No. 7, CDR2 comprising the amino acid sequence of SEQ ID No. 9 and CDR3 comprising the amino acid sequence of SEQ ID No. 11, while the second anti-HER2 antibody is a biotin-bound antibody whose heavy chain variable region comprises CDR1 comprising the amino acid sequence of SEQ ID No. 2, CDR2 comprising the amino acid sequence of SEQ ID No. 4 and CDR3 comprising the amino acid sequence of SEQ ID No. 6, and the light chain variable region of said second antibody comprises CDR1 comprises the amino acid sequence of SEQ ID No. 8, CDR2 comprises the amino acid sequence of SEQ ID No. 10 and CDR3 comprises the amino acid sequence of SEQ ID No. 12.

In one embodiment, the heavy chain variable region of the antibody according to the invention comprises CDR1 having the amino acid sequence of GYSFTGYN (SEQ ID No. 1), CDR2 having the amino acid sequence of IDPNYGT (SEQ ID No. 3) and CDR3 having the amino acid sequence of ARRKVNYEAWFAF (SEQ ID No. 5). In contrast, the light chain variable region of said antibody comprises CDR1 with the amino acid sequence of ESVDSYGNSF (SEQ ID No. 7), CDR2 with the amino acid sequence of RAS (SEQ ID No. 9) and CDR3 with the amino acid sequence of QQSNEDPFT (SEQ ID No. 11).

In another embodiment, the heavy chain variable region of the antibody according to the invention comprises CDR1 having the amino acid sequence of GYSFTDYN (SEQ ID No. 2), CDR2 having the amino acid sequence of IDHYNGNT (SEQ ID No. 4) and CDR3 having the amino acid sequence of ARHYDYGAMDY (SEQ ID No. 6). In contrast, the light chain variable region of said antibody comprises CDR1 with the amino acid sequence of QNVGTN (SEQ ID No. 8), CDR2 with the amino acid sequence of SAS (SEQ ID No. 10) and CDR3 with the amino acid sequence of QHYYIYPLT (SEQ ID No. 12).

In one embodiment, the first anti-HER2 antibody comprises a heavy chain variable region comprising the amino acid sequence of SEQ ID No. 13 with either 0, 1, 2, 3, 4 or 5 insertions, deletions of substitutions or amino acid additions, and comprises a light chain variable region comprising the amino acid sequence of SEQ ID 15 with either 0, 1, 2, 3, 4 or 5 insertions, deletions of substitutions or amino acid additions.

In another embodiment, said first antibody comprises a heavy chain variable region consisting of the amino acid sequence of SEQ ID No. 13 with either 0, 1, 2, 3, 4 or 5 insertions, deletions of substitutions or amino acid additions, and the light chain variable region consists of the amino acid sequence of SEQ ID 15 with either 0, 1, 2, 3, 4 or 5 insertions, deletions of substitutions or amino acid additions.

In another embodiment, the second biotin-bound antibody heavy chain variable region comprises the amino acid sequence of SEQ ID No. 14 with either 0, 1, 2, 3, 4 or 5 insertions, deletions of substitutions or amino acid additions and comprises light chain variable region comprising the amino acid sequence of SEQ ID 16 with either 0, 1, 2, 3, 4 or 5 insertions, deletions of substitutions or amino acid additions.

In another embodiment, said second anti-HER2 antibody according to the invention comprises a heavy chain variable region consisting of the amino acid sequence of SEQ ID No. 14 with either 0, 1, 2, 3, 4 or 5 insertions, deletions of substitutions or amino acid additions and the light chain variable region consisting of the amino acid sequence of SEQ ID 16 with 0, 1, 2, 3, 4 or 5 insertions, deletions of substitutions or amino acid additions.

In another embodiment, the heavy chain variable region of the first antibody is the amino acid sequence of SEQ ID No. 13 and its light chain variable region is the amino acid sequence of SEQ ID No. 15.

In another embodiment, the heavy chain variable region of the biotin-bound second antibody is the amino acid sequence of SEQ ID No. 14 and its light chain variable region is the amino acid sequence of SEQ ID No. 16.

In another embodiment, the heavy chain of the first antibody is the amino acid sequence of SEQ ID No. 29 or SEQ ID No. 29 with either 0, 1, 2, 3, 4, 5, 6, 7, 8, 9 or 10 amino acid insertions, deletions substitutions or additions.

In another embodiment, the heavy chain of the second antibody is the amino acid sequence of SEQ ID No. 30 or SEQ ID No. 30 with either 0, 1, 2, 3, 4, 5, 6, 7, 8, 9 or 10 amino acid insertions, deletions substitutions or additions.

In another embodiment, the light chain of the first antibody is the amino acid sequence of SEQ ID No. 31 or SEQ ID No. 31 with either 0, 1, 2, 3, 4, 5, 6, 7, 8, 9 or 10 amino acid insertions, deletions substitutions or additions.

In another embodiment, the light chain of the second antibody is the amino acid sequence of SEQ ID No. 32 or SEQ ID No. 32 with either 0, 1, 2, 3, 4, 5, 6, 7, 8, 9 or 10 amino acid insertions, deletions substitutions or additions.

In a further aspect, the invention relates to the use of antibody/antibodies according to the invention to produce antibody-drug conjugates (ADCs).

*Antibody-drug conjugates* (ADCs) are considered as alternative to chemotherapy, next-generation cancer therapy based on improving the transport of anticancer drugs to target cells in the patient's body. ADCs are conjugates of monoclonal antibodies directed against a tumour cell antigen and a biologically active substance with cytotoxic activity. It is therefore an individual class of biopharmaceutical drugs for targeted cancer therapy with high selectivity. By binding to the antigen, the antibody delivers a cytotoxic substance in the area of the cancer cell, whose negative effects on healthy cells throughout the body (as in standard chemotherapy) are limited and concentrated in the location of the cancer cells, remaining effective at lower drug concentrations. The first ADCs were approved for use in the US by the FDA in 2001 and dozens of formulations have been used in therapy since then.

ADCs function is to interfere with the normal functioning of cells and, therefore, their action concerns basic cellular processes. There are many variations of ADCs, depending on the type and form of the antibody recognising the antigen, e.g., diabody, Fab, scFV, bicyclic peptides, as well as the number of drug molecules carried simultaneously or their type, including nanocarriers, liposomes and polymeric molecules. ADC therapy is popular, not least because of the widespread use of its components, i.e., chemotherapeutics and monoclonal antibodies in therapy. Conjugates therefore remain a slightly improved, more selective and more effective form of a well-known and long-standing cancer therapy.

In ADC conjugates, the anti-HER2 antibodies according to the invention undergo covalent binding to the drug molecule via a linker.

Whereby, in one embodiment, the antibody according to the invention used comprises the heavy chain variable region comprising CDR1 having the amino acid sequence of GYSFTGYN (SEQ ID No. 1), CDR2 having the amino acid sequence of IDPNYGT (SEQ ID No. 3) and CDR3 having the amino acid sequence of ARRKVNYEAWFAF (SEQ ID No. 5). Wherein, the light chain variable region of said antibody comprises CDR1 with the amino acid sequence of ESVDSYGNSF (SEQ ID No. 7), CDR2 with the amino acid sequence of RAS (SEQ ID No. 9) and CDR3 with the amino acid sequence of QQSNEDPFT (SEQ ID No. 11).

In another embodiment, the antibody according to the invention used, comprises a heavy chain variable region comprising CDR1 having the amino acid sequence of GYSFTDYN (SEQ ID No. 2), CDR2 having the amino acid sequence of IDHYNGNT (SEQ ID No. 4) and CDR3 having the amino acid sequence of ARHYDYGAMDY (SEQ ID No. 6). Wherein, the light chain variable region of said antibody comprises CDR1 with the amino acid sequence of QNVGTN (SEQ ID No. 8), CDR2 with the amino acid sequence of SAS (SEQ ID No. 10) and CDR3 with the amino acid sequence of QHYYIYPLT (SEQ ID No. 12).

In one embodiment, the anti-HER2 antibody according to the invention used comprises a heavy chain variable region comprising the amino acid sequence of SEQ ID No. 13 with either 0, 1, 2, 3, 4 or 5 insertions, deletions of substitutions or amino acid additions, and the light chain variable region of said antibody comprises the amino acid sequence of SEQ ID No. 15 with either 0, 1, 2, 3, 4 or 5 insertions, deletions of substitutions or amino acid additions.

In another embodiment, the anti-HER2 antibody according to the invention used comprises a heavy chain variable region consisting of the amino acid sequence of SEQ ID No. 13 with either 0, 1, 2, 3, 4 or 5 insertions, deletions of substitutions or amino acid additions and wherein a light chain variable region consists of the amino acid sequence of SEQ ID No. 15 with 0, 1, 2, 3, 4 or 5 insertions, deletions of substitutions or amino acid additions is used.

In another embodiment, the anti-HER2 antibody according to the invention comprises a heavy chain variable region comprising the amino acid sequence of SEQ ID No. 14 with either 0, 1, 2, 3, 4 or 5 insertions, deletions of substitutions or amino acid additions, and the light chain variable region comprising the amino acid sequence of SEQ ID No. 16 with either 0, 1, 2, 3, 4 or 5 insertions, deletions of substitutions or amino acid additions, is used.

In another embodiment, the anti-HER2 antibody according to the invention comprises a heavy chain variable region consisting of the amino acid sequence of SEQ ID No. 14 with either 0, 1, 2, 3, 4 or 5 insertions, deletions of substitutions or amino acid additions and said antibody comprises a light chain variable region consisting of the amino acid sequence of SEQ ID No. 16 with either 0, 1, 2, 3, 4 or 5 insertions, deletions of substitutions or amino acid additions.

In another embodiment, the heavy chain variable region of the used antibody according to the invention consists of the amino acid sequence of SEQ ID No. 13 and its light chain variable region consists of the amino acid sequence of SEQ ID No. 15.

In another embodiment, the heavy chain variable region of the used antibody according to the invention consists of the amino acid sequence of SEQ ID No. 14 and its light chain variable region consists of the amino acid sequence of SEQ ID No. 16.

In another embodiment, the heavy chain of the used antibody according to the invention consists of the amino acid sequence of SEQ ID No. 29 or SEQ ID No. 29 with either 0, 1, 2, 3, 4, 5, 6, 7, 8, 9 or 10 amino acid insertions, deletions, substitutions or additions.

In another embodiment, the heavy chain of the used antibody according to the invention consists of the amino acid sequence of SEQ ID No. 30 or SEQ ID No. 30 with either 0, 1, 2, 3, 4, 5, 6, 7, 8, 9 or 10 amino acid insertions, deletions, substitutions or additions.

In another embodiment, the light chain of the used antibody according to the invention consists of the amino acid sequence of SEQ ID No. 31 or SEQ ID No. 31 with either 0, 1, 2, 3, 4, 5, 6, 7, 8, 9 or 10 amino acid insertions, deletions, substitutions or additions.

In another embodiment, the light chain of the used antibody according to the invention consists of the amino acid sequence of SEQ ID No. 32 or SEQ ID No. 32 with either 0, 1, 2, 3, 4, 5, 6, 7, 8, 9 or 10 amino acid insertions, deletions, substitutions or additions.

In another embodiment, the heavy chain of the used antibody according to the invention consists of the amino acid sequence of SEQ ID No. 29 or SEQ ID No. 29 with either 0, 1, 2, 3, 4, 5, 6, 7, 8, 9 or 10 insertions, substitution deletion or amino acid addition, while the light chain consists of an amino acid sequence of SEQ ID No. 31 or SEQ ID No. 31 with either 0, 1, 2, 3, 4, 5, 6, 7, 8, 9 or 10 insertions, substitution deletions or amino acid additions.

In another embodiment, the heavy chain of the antibody according to the invention used consists of the amino acid sequence of SEQ ID No. 30 or SEQ ID No. 30 with either 0, 1, 2, 3, 4, 5, 6, 7, 8, 9 or 10 insertions, substitution deletion or amino acid addition, while the light chain consists of the amino acid sequence of SEQ ID No. 32 or SEQ ID No. 32 with either 0, 1, 2, 3, 4, 5, 6, 7, 8, 9 or 10 insertions, substitution deletions or amino acid additions.

In a preferable embodiment, the anticancer drugs used in the antibody conjugates according to the invention are selected from a group including in particular, but not limited to: microtubulin inhibitor, auristatin A (MMAE), DNA-binding calicheamycin, topoisomerase 1 inhibitor SN-38, glucocorticoid receptor modulator GRM.

In a preferable embodiment, in order to increase the efficiency of transport in the human body while reducing the risk of removal by the patient's immune system, the anti-HER2 antibodies according to the invention are humanised. In such process, an antibody hybrid is created in which the base is formed by a human antibody, while the variable regions - which specifically recognise the antigen - are derived from an antibody of a different species. The use of the anti-HER2 antibody hypervariable region sequences, which are the subject of the present invention in humanised bio-drug molecules, will enable the preservation of HER2 binding specificity, comparable to that of tested antibodies (murine antibodies), and the risk of recognition by the patient's immune system as a foreign molecule, leading to its removal, is reduced. The innovation of such use of the unique variable regions of the presented antibodies would be based on their specificity towards HER2 and the possibility of selecting the appropriate type of conjugated cytotoxin.

In a further aspect, the invention relates to the use of nucleotide sequences encoding the variable regions of antibodies according to the invention for the production of CAR-T.

CAR-T (*chimeric antigen receptor T-cell*) therapy is an innovative type of cellular immunotherapy using chimeric (modified) T cells. The essence of CAR-T mode of action is targeted redirection of lymphocytes to a selected molecular target in order to stimulate their anti-cancer role, through a combination of two types of specific immune response - cytotoxic from T cells and humoral based on antibody specificity. The CAR-T therapy was approved by the Food and Drug Administration (FDA) for use in the US in 2017 and is also available in Poland for the treatment of acute lymphoblastic leukaemia from 2021.

The CAR-T therapy is based on a chimeric antigen receptor (CAR), modified by genetic engineering methods and designed to recognise the indicated therapeutic target. The chimeric receptor consists of an extracellular part that recognises the target antigen (scFv, or a single-chain variable fragment) via an antibody light chain, a separation domain, a trans-membrane domain and an intracellular signalling domain. Once antigen is bound, activation of the T lymphocyte occurs in a system independent of tissue compatibility proteins, leading to T cell stimulation, cytolysis and cytokine secretion leading to elimination of the target cell. Depending on the amount and type of signalling molecule (e.g., CD3ξ), a distinction is made between first-, second-, third- or fourth-generation CAR-T.

In practice, T lymphocytes are collected from patients and then isolated from the blood and subjected to modification by genetic engineering and molecular biology methods. The modified chimeric lymphocytes, after multiplication in vitro, are returned to the patient in the form of an infusion. Thanks to their specific affinity for the target antigen, CAR-T lymphocytes find tumour cells in the patient's body and direct an immune response against them. This therapy has the advantage of recognising antigen from tumour cells and activating the lymphocytes independently of *major histocompatibility complex* (MHC) restrictions. This is particularly important in the context of the frequent loss of MHC-associated antigen presentation in tumour cells, as a defence mechanism against the immune system. It is estimated that complete remission of chronic lymphocytic leukaemia (CLL) and non-Hodgkin's lymphoma (NHL) is showed by 35-70% of patients undergoing treatment. Great hopes are placed on treating patients who do not respond to any other therapies. In this group of acute lymphoblastic leukaemia patients, it is estimated that 9 out of 10 therapies end in remission.

The variable regions of the anti-HER2 monoclonal antibodies that are the subject of the present invention, thanks to their antigen-binding specificity and proven lack of cytotoxicity, could potentially find their application in personalised CAR-T therapy.

In some embodiments, the nucleic acid molecule encoding the variable region of the anti-HER2 antibody according to the invention in the integrating vector can be introduced into the T lymphocyte by electroporation methods, using gammaretroviral vectors, lentiviral vectors or based on transposon/transposase systems, ensuring stable integration into the genome of T lymphocytes and their progenitor cells.

In a preferable embodiment, the nucleic acid molecule encodes the variable region of the anti-HER2 antibody according to the invention, which is selected from the group comprising SEQ No. 17, SEQ No. 18, SEQ No. 19, SEQ No. 20, SEQ No. 21, SEQ No. 22, SEQ No. 23, SEQ No. 24, SEQ No. 25, SEQ No. 26, SEQ No. 27, SEQ No. 28.

The HER2 antigen highly expressed on the surface of tumour cells, being recognised by the chimeric CAR-T receptor thanks to a fragment of the anti-HER2 antibody light chain variable sequence, could potentially lead to activation of the immune response and removal of such cells from the patient's body. As HER2 is also localised in small amounts in healthy cells, it would be necessary to use iCAR (*inhibitory chimeric antigen receptors*) technology to protect the patient's body from the effects of an autoimmune and immune response directed against healthy cells. The iCAR technology uses a typical CAR receptor (a-CAR) directed against a tumour cell surface antigen, which may also be present in healthy cells, and the iCAR protective system, exploiting the fact that certain antigens are lost in tumour cells due to a chromosomal *loss-of heterozygosity* (LOH) mechanism. In this case, healthy cells expressing the iCAR antigen (e.g., HLA-A2 or programmed cell death protein 1, PD-1) are protected, while cancer cells lacking this antigen are killed. Such a two-step protection system limits the cytotoxicity of CAR-T against healthy cells and carries enormous potential for use in solid tumours.

The invention is illustrated in the examples and in the figures wherein:

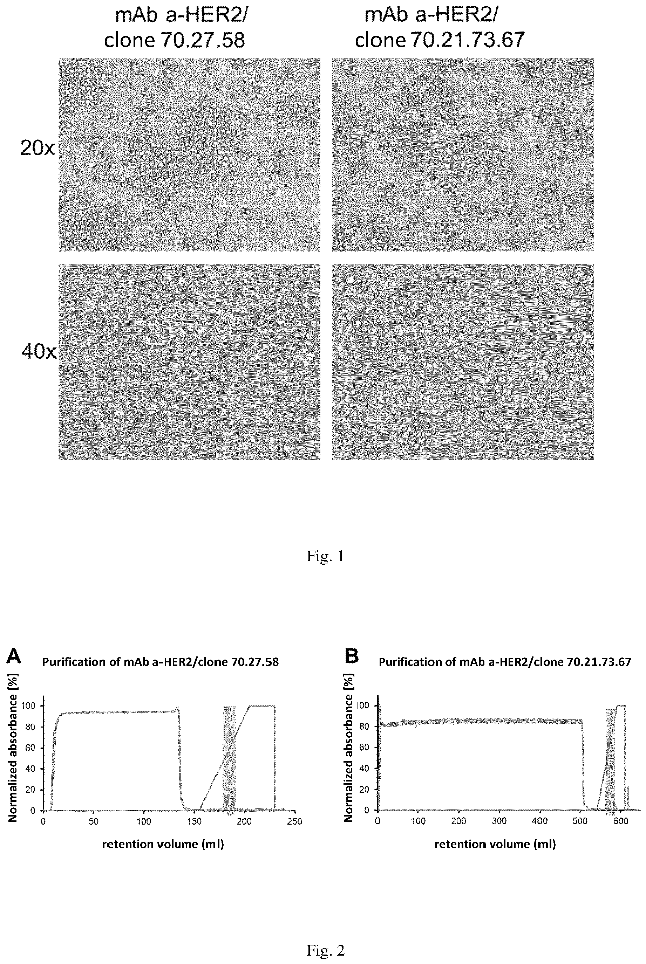

fig. 1 shows microscopic images of hybridoma cells producing antibodies according to the invention in the first variant labelled as mAb anti-human-HER2/70.27.58 i.e., clone 58 and in the second variant labelled as mAb anti-human-HER2/70.21.73.67 i.e., clone 67 at 20- and 40-fold magnification, the images were taken using a Delta Optical IB-100 inverted microscope;
fig. 2 shows representative chromatograms from purification of mAb murine antibodies: anti-human-HER2/70.27.58, i.e., clone 58 (A) and anti-human-HER2/70.21.73.67, i.e., clone 67 (B), wherein orange line represents the absorbance reading at 280 nm, corresponding to the presence of protein in solution, blue line represents the concentration of Buffer B upon gradient elution of the antibody from the HiTrapTM Protein A HP column, the antibodies are eluted from the column at -50% Buffer B concentration, and orange block indicates the peaks corresponding to the antibodies being purified;

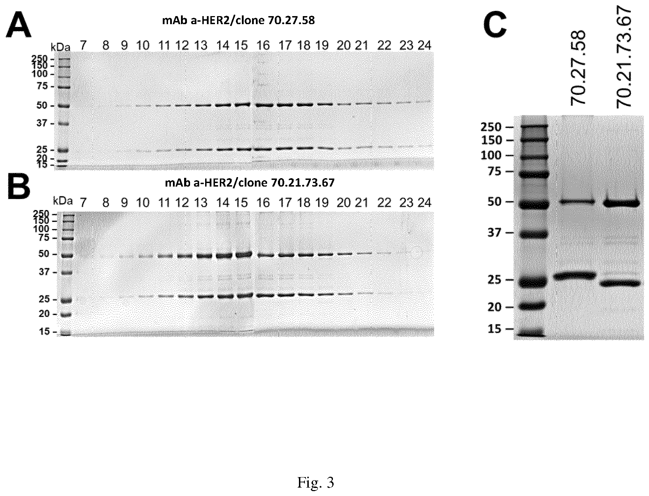

fig. 3 shows an evaluation of the protein band patterns of murine antibodies: anti-human HER2/70.27.58 i.e., clone 58 (A) and anti-human HER2/70.21.73.67 i.e., clone 67 (B) in fractions purified on a protein A column under denaturing and reducing conditions, wherein the ~50 kDa band corresponds to the heavy chain and the ~25 kDa band corresponds to the light chain, and (C) shows a comparison of the protein band profile for purified murine monoclonal antibodies anti-human HER2/70.27.58 (clone 58) and anti-human/70.21.73.67 (clone 67) by SDS-PAGE, with 2.0 µg of antibody per well applied to the gel and analysed under reducing conditions;

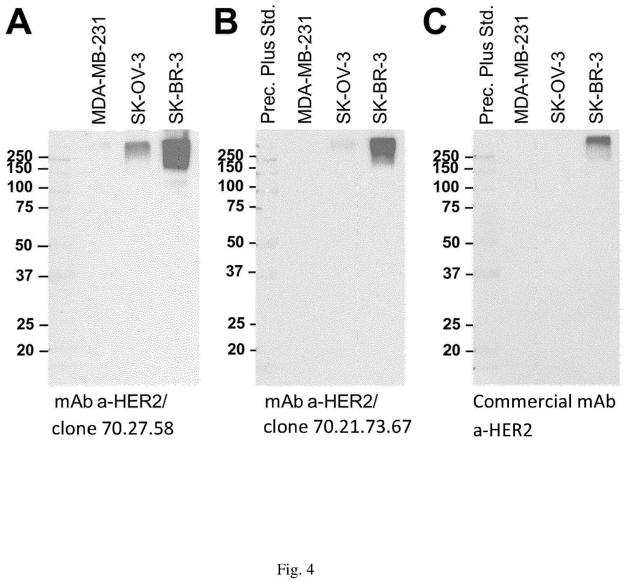

fig. 4 shows HER2 levels in cell lysates of the MDA-MB-231, SK-OV-3 and SK-BR-3 cell lines subjected to Western blot detection using (A) anti-HER2/70.27.58 - clone 58, (B) anti-HER2/70.21.73.67 - clone 67 and (C) a commercially available murine monoclonal anti-HER2 antibody (Abeam, ab16901), with cell lysates comprising 40 µg of protein analysed and all membranes were exposed for an equivalent time of 43 sec;

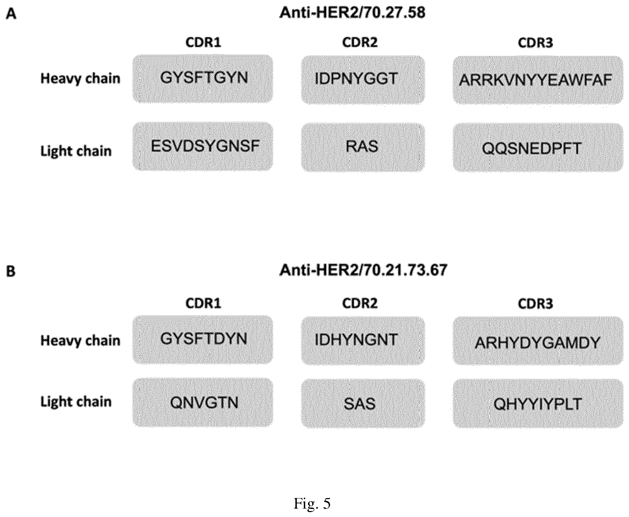

fig. 5 shows the amino acid sequences of the CDR1-3 antibodies according to the invention, wherein (A) shows the CDR1-3 sequences of antibody according to the first varia-t - i.e., the CDR1-3 of mAb murine anti-human HER2/70.27.58 (clone 58), and (B) shows the CDR1-3 sequences of antibody according to the second varia-t - i.e., the CDR1-3 of mAb murine anti-human HER2/70.21.73.67 (clone 67);

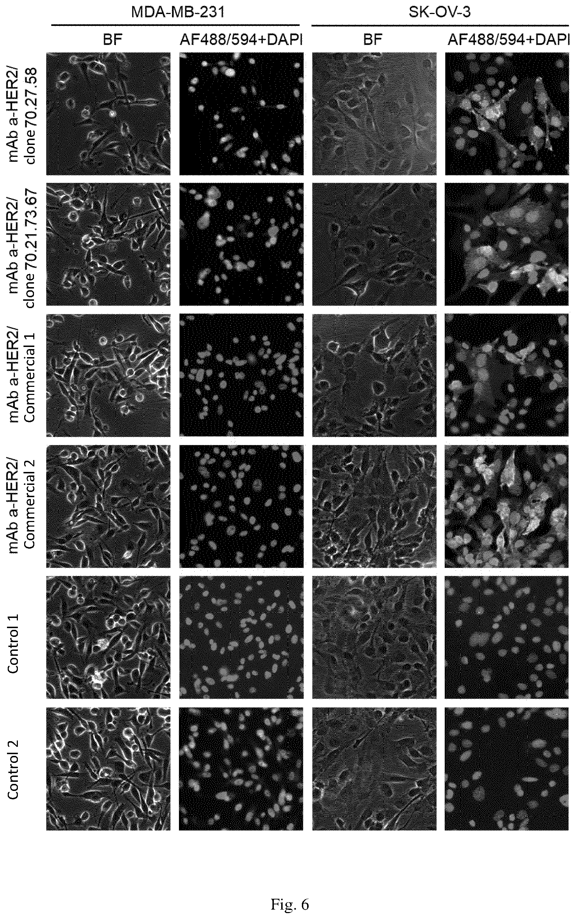

fig. 6 shows a comparison of HER2 antigen detection by antibodies in fixed cells of MDA-MB-231 (HER2-negative) and SK-OV-3 (HER2-positive) cell lines, wherein the following antibodies were used as primary antibodies: anti-HER2/70.27.58 (clone 58), anti-HER2/70.21.73.67 (clone 67), and 2 commercially available anti-HER2 monoclonal antibodies (mAb rabbit anti-HER2/ ab194979 (Abeam) - designated as commercial 1 and murine anti-HER2/ MA1_12691 monoclonal antibody (Thermo Fisher) - designated as commercial 2) were reacted with fluorophore-conjugated II-strand antibodies (Alexa488 for the murine antibody or Alexa594 for the rabbit antibody), While cell nuclei were stained with DAPI, cells were visualised using a Nikon Eclipse Ti2-U bright field (BF) microscope equipped with a Nikon Eclipse Ti2-U, Bright field (BF) microscope equipped with a Plan Fluor 10x objective, and the negative control was anti-rabbit (control 1) or anti-murine (control 2) antibody II alone;

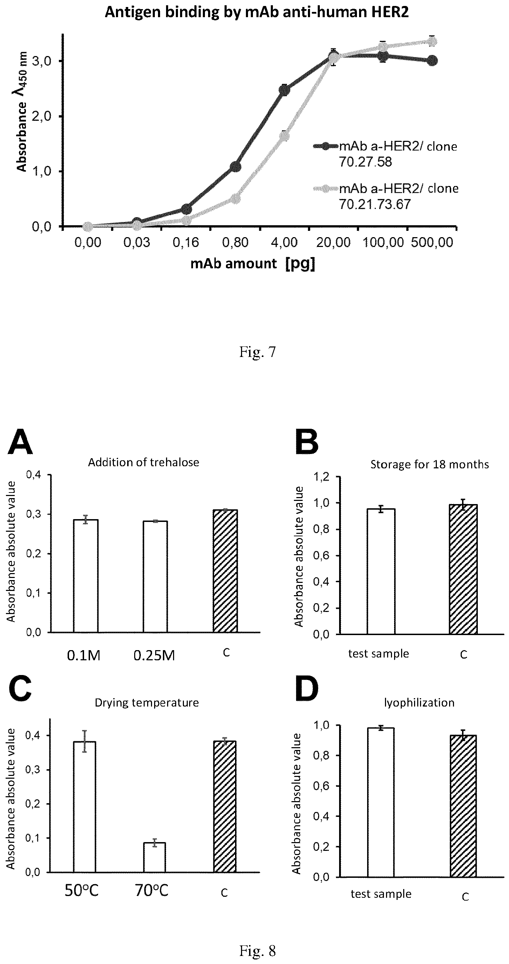

fig. 7 shows a graph comparing the HER2 antigen binding capacity of antibodies according to the invention labelled as mAb murine anti-human HER2/ 70.27.58 - clone 58 (antibody according to the first variant) and anti-human/70.21.73.67 - clone 67 (antibody according to the second variant);
fig. 8 shows the stability analysis of the mAb murine anti-human HER2/ 70.27.58. A - clone 58, wherein: (A) shows the HER2 antigen binding capacity of the antibody dried at 37 °C in the presence of 0.1 and 0.25 M trehalose, with the HER2 antigen recognition capacity analysed 24 h after drying, (B) shows analysis of antigen binding by an antibody stored in PPBS solution with 50% glycerol at -80 °C for 18 months, (C) shows a comparison of the effect of drying temperature on antibody stability, wherein the antibody dried at 37-50 °C remains fully stable and retains full antigen binding capacity after re-hydration, wherein denaturation of the antibody only occurs at extreme temperatures such as 70 °C, D) shows HER2 antigen-binding capacity of the freeze-dried and re-hydrated antibody after a period of 7 days relative to a non-freeze-dried control, wherein the control in all performed experiment was an equivalent amount of antibody without the additional substances added, untreated and stored at 40°C until analysis;

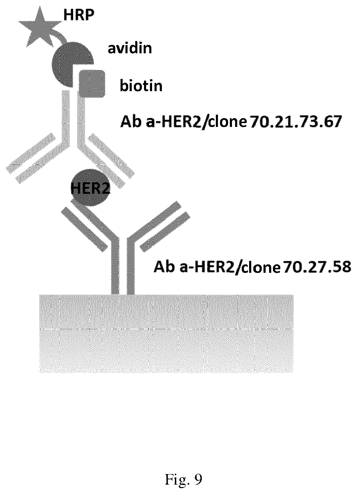

fig. 9 shows a diagram of the "sandwich" of the immunoenzymatic assay according to the invention;

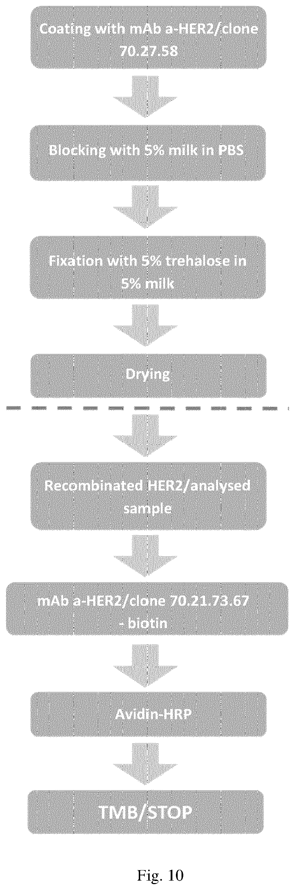

fig. 10 shows a block diagram of the steps of preparing and carrying out the method according to the invention using an immunoenzymatic assay, wherein the dotted line indicates the step where plate coating can be stopped after drying and said plate stored afterwards for up to 4 months under controlled conditions;

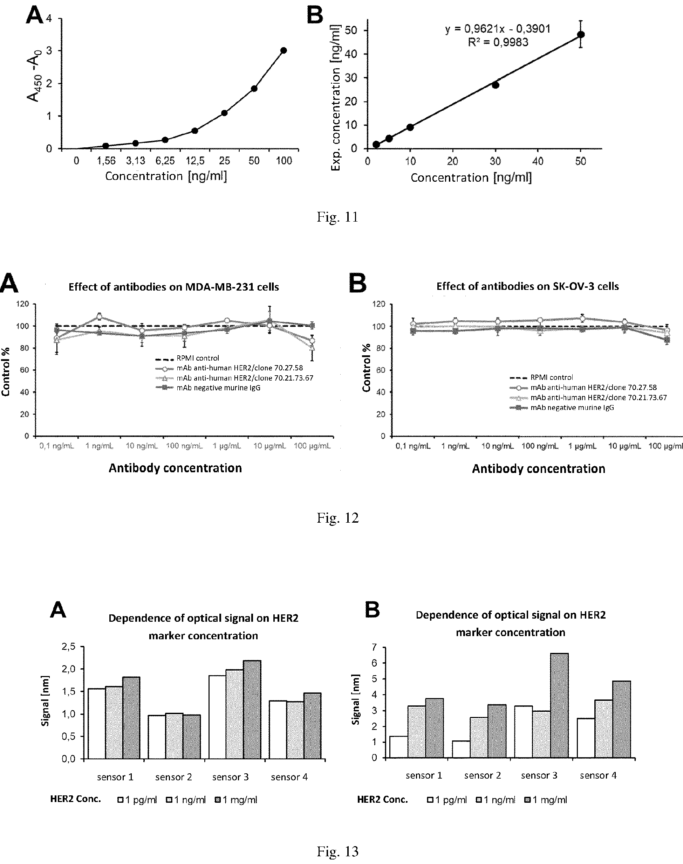

fig. 11 shows a comparison of the detection of increasing concentrations of HER2 antigen [ng/ml] in an ELISA immunoenzymatic assay, wherein (A) shows analysis performed using a coated plate and stored for 4 months; and (B) shows a comparison of experimental measurement for samples with known analyte concentrations in the range 2-50 ng/ml;
fig. 12 shows increasing concentrations of anti-human HER2/clone 70.27.58 and anti-human HER2/clone 70.21.73.67 antibodies on tumour cells of the MDA-MB-231 (A) and SK-OV-3 (B) cell lines, wherein the data obtained in the MTT assay were compared with the RPMI substrate control (without added antibodies) and compared with the effect of murine IgG class antibodies obtained from non-immunized mice;
fig. 13 shows measurements of the dependence of the optical signal on the concentration of the HER2 marker carried out on optoelectronic biosensors coated with (A) an F(ab')2 fragment derived from a murine anti-human HER2 antibodies according to the invention or (B) a full murine anti-human HER2 antibodies according to the invention, wherein the results shows measurements for 4 individual sensors, and for each sensor three concentrations of HER2 were tested consecutively: 1 pg/ml, 1 ng/ml and 1 mg/ml and wavelength changes were recorded;

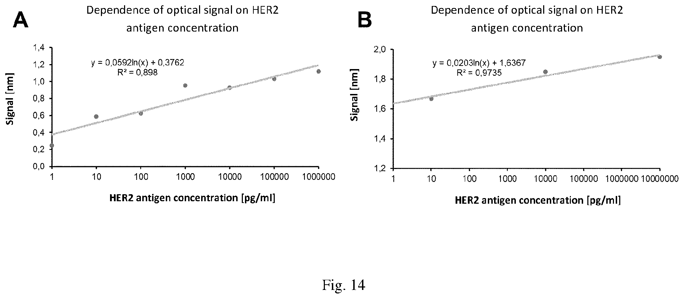

fig. 14 shows a graph of the dependence of the wavelength minimum shift (in nanometres) on the concentration of antigen in which the sensor is immersed, wherein (A) shows a graph comprising the averaged measurements collected for: 72 sensors subjected to the analysis using the full anti-HER2 antibodies according to the invention and (B) 24 sensors subjected to the analysis using the F(ab')2 anti-HER2 antibodies according to the invention.

The invention is presented in following examples, and all assays and experimental procedures described below were carried out using commercially available test kits, reagents and apparatus, following the manufacturer's instructions, unless specifically indicated otherwise. All test parameters were measured using standard, well-known methods used in the field of present invention.

However, the following examples serve to illustrate the present invention and should not be considered as a limitation of the scope of invention.

### Example 1.

In this exemplary embodiment, the properties of monoclonal anti-HER2 antibodies according to invention with high specificity towards HER2 were analysed, wherein tested antibodies were designated as:
- mAb anti-human-HER2/70.27.58 - clone 58,
- mAb anti-human-HER2/70.21.73.67 - clone 67.

Wherein, the mAb anti-human-HER2/clone 58 is an antibody having a heavy chain consisting of the amino acid sequence of SEQ ID No. 29 and having a light chain consisting of the amino acid sequence of SEQ ID No. 31. The nucleic acid molecule encoding the heavy chain of this antibody comprises the nucleotide sequence of SEQ ID No. 33 and the nucleic acid molecule encoding the light chain of this antibody comprises the nucleotide sequence of SEQ ID No. 35.

On the other hand, the mAb anti-human-HER2/clone 67 is an antibody whose heavy chain consists of the amino acid sequence of SEQ ID No. 30 and whose light chain consists of the amino acid sequence of SEQ ID No. 32. The nucleic acid molecule encoding the heavy chain of this antibody comprises the nucleotide sequence of SEQ ID No. 34, and the nucleic acid molecule encoding the light chain of this antibody comprises the nucleotide sequence of SEQ ID No. 36.

### Obtaining antibodies according to the invention

In this exemplary embodiment, monoclonal antibodies with high specificity towards HER2 (i.e., mAb anti-human-HER2/clone 58 and mAb anti-human-HER2/clone 67) were obtained by immunising Balb/c mice with the respective antigen. A fusion of antibody-producing murine B cells with immortalised murine Sp2/O-Ag14 myeloma cells yielded hybridoma cells. Under controlled laboratory conditions, these cells retained the immunological capacity of short-lived B cells, while maintaining an unlimited capacity to divide. After appropriate selection, lines derived from single fusion cells produced antibodies identical in terms of the recognised epitope.

The two, hybridoma cell lines described herein produced monoclonal mAb anti-human HER2 antibodies, designated as clone 58 and clone 67 respectively, directed against distinct fragments of the HER2 antigen.

Each hybridoma cell culture was carried out at 37 °C in an atmosphere of 5% CO₂ according to an established protocol. Briefly, adherent and suspension hybridoma cells were initially cultured in rich DMEM (Dulbecco's Modified Eagle Medium) supplemented with 10% FBS (fetal bovine serum) until full confluence is achieved, i.e., full coverage of the surface of the culture bottle or plate with a single layer. Cells in culture maintained a viability of 70-80%. The cells were then passaged and the medium replaced with Panserin H4000 (PAN-Biotech, P04-714001) completely devoid of protein components. The highest efficiency of antibody production was achieved under concentrated culture conditions; therefore, cultures were continued in one bottle with regular replacement of the medium with fresh medium. Representative images of cells in culture for hybridomas producing the mAb murine anti-humanHER2/clone 58 and clone 67 at two magnifications are shown in Fig. 1.

The medium collected from the hybridoma cells, the so-called post culture medium, is the source of the antibodies. Using affinity chromatography methods, the antibodies were then separated from the other components of the medium. In this exemplary embodiment, for each antibody, purification is carried out on a column containing Protein A (HiTrap^{®} Protein A High Performance, Cytiva 17-0403-01) using an NGC liquid chromatography system (NGC Medium-Pressure Chromatography System; Bio-Rad). For this purpose, an appropriately prepared Protein A column is equilibrated with binding buffer (Buffer A: 0.2 M boric acid, 0.02 M sodium phosphate, 0.02 M sodium citrate dihydrate, 1.0 M sodium sulphate, pH 9.0). The culture medium was afterwards applied to the column and the fraction of proteins not bound to the column passed through the bed and was visible as an increase in absorbance signal at 280 nm on the chromatogram. After all of the culture medium has been applied to the column and washed with binding buffer, only the anti-human HER2 antibodies according to the invention remained bound to the column. They were released from the column in a gradient of buffer A and elution buffer B (Buffer B: 0.02 M sodium citrate dihydrate, 0.1 M sodium chloride, pH 2.5). The antibody is eluted from the column at -50% buffer B and is visible on the chromatogram as a symmetrical peak. An example chromatogram from purification of anti-human HER2 antibody/clone 58 (A) and clone 67 (B) is shown in Fig. 2, and the peaks corresponding to the antibodies are indicated with blocks.

Antibodies are eluted into a series of tubes as successive fractions and immediately neutralised with 3 M Tris-HCl pH 8.8. The presence of protein in the individual fractions is confirmed by Nanodrop protein concentration measurements and the band pattern was analysed by SDS-PAGE (as indicated in Fig. 3). In this exemplary embodiment, fractions containing the highest protein concentrations were combined and subjected to phosphate buffered saline (PBS) exchange using Amicon centrifuge filters with a cut-off mass of 30 kDa. The production yields from continuous culture of hybridoma cells were 3.98 mg ± 2.99 and 2.92 mg ± 1.6 of protein per litre of culture medium for mAb antibodies anti-human HER2/clone 58 and anti-human HER2/clone 67, respectively.

The final samples of the mAb murine anti-human HER2/clone 58 and anti-human/clone 67 antibodies exhibited a high reproducibility and purity of preparation. Under non-reducing conditions, single bands of -180 kDa were visible on SDS-PAGE, while under reducing conditions they broke down into heavy chains of ~50 kDa and light chains of 25 kDa (Fig. 3C). The CDR1-3 sequences of the resulting antibodies are shown in fig. 5.

### Example 2.

### Confirmation of the specificity of the antibodies according to the invention against the HER2 antigen

The specificity of the antibodies according to Example 1 against the HER2 antigen was confirmed in both cell lysates by Western blot (Fig. 4) and whole cells analysed by immunofluorescence (Fig. 6).

Performed analysis showed that the anti-HER2 antibodies according to the invention correctly detected the antigen (a band at approximately > 250 kDa) in SK-BR-3 (ATCC^{®} HTB-30^{™}) and SK-OV-3 (ATCC^{®} HTB-77^{™}) cell lysates, as well as the recombinant HER2 ECD domain, while lacking signal in MDA-MB-231 (ATCC^{®} HTB-26^{™}) cell lysates (Fig. 4). A commercial monoclonal murine anti-HER2 antibody (Abeam, ab16901) was used as a control.

The antibodies according to the invention also detect the HER2 antigen in cells when detection is carried out by immunofluorescence methods. For comparison, the analysis was performed on two commercially available anti-HER2 monoclonal antibodies.

The following primary antibodies were used for analysis: anti-HER2/clone 70.27.58 (clone 58), anti-HER2/clone 70.21.73.67 (clone 67), and 2 commercially available anti-HER2 monoclonal antibodies (mAb rabbit anti-HER2/ ab194979 (Abeam) - commercial 1 and monoclonal murine anti-HER2/ MA1_12691 antibody (Thermo Fisher) - commercial 2), which were reacted with fluorophore-conjugated II-strand antibodies (Alexa488 for the murine antibody or Alexa594 for the rabbit antibody). Cell nuclei were stained with DAPI. Cells were visualised using a Nikon Eclipse Ti2-U bright field (BF) microscope equipped with a Plan Fluor 10x objective. Negative controls were anti-rabbit (control 1) or anti-murine (control 2) II-antibodies alone.

The results are shown in fig. 6, and the performed analysis showed that the HER2 protein signal is detected in the SK-OV-3 line (HER2-positive) and absent in the MDA-MB-231 negative control cells (HER2-negative). Furthermore, the analysis showed that the localisation and strength of the signal is comparable in SK-OV-3 cells.

### Example 3.

### Antigen binding strength determination for clone 58 and 67 antibodies according to the invention

The antibodies according to the invention specifically recognise the HER2 antigen through the unique CDR1-3 sequence, as schematically shown in fig. 5.

Determination of the antigen binding strength of mAb anti-human HER2/70.27.58 - clone 58 and anti-human HER2/70.21.73.67 - clone 67 antibodies according to example 1 was carried out according to the following protocol. Fig. 7 shows a comparison of the HER2 antigen binding capacity of the mAb murine anti-human HER2/clone 58 and anti-human/clone 67 antibodies.

The strength of antigen binding for both antibody clones was confirmed in vitro by determining the ED50 value, i.e., half of the effective dose. This value is used as a parameter to illustrate the pharmacokinetics of antibodies and drugs. For the mAb murine anti-human HER2/clone 58 and anti-human HER2/clone 67 antibodies, these values were determined at 0.0922 nM and 0.2347 nM, respectively, representing strong binding.

The antigen-binding strength, which remains the main parameter characterising the antibodies according to the invention, and the specificity, which allows detection of only the true antigen, without cross-reactivity and detection of so-called off-targets, constitute their uniqueness.

### Stability determination of antibodies according to the invention

### A) Stability study of mAb anti-human HER2/70.27.58 antibody - clone 58

To assess the stability of the mAb anti-human HER2/70.27.58 - clone 58 antibody, following evaluation were conducted:
a) evaluation of HER2 antigen binding capacity of the antibody dried at 37 °C in the presence of 0.1 and 0.25 M trehalose, wherein the ability to recognise the HER2 antigen was analysed 24 h after drying;
b) evaluation of antigen binding by an antibody stored in PBS solution with 50% glycerol at -80 °C for 18 months when stored at -80°C;
c) evaluation of the effect of drying temperature on antibody stability by comparing the antigen-binding capacity after re-hydration of antibodies dried at 37-50 °C and at 70 °C;
d) evaluation of HER2 antigen binding by a lyophilised and re-hydrated antibody after a period of 7 days relative to a non-lyophilised control sample.

Comparative assessments were made against a control sample, which was an equivalent amount of the tested antibody without additional substances added, untreated and stored at 4 °C until analysis.

Performed analysis showed that the HER2/70.27.58 - clone 58 antibody according to the invention has high stability and functionality under extreme conditions. This antibody can be dried at 37 °C in an immobilised form on a plate with the addition of 100-250 mM trehalose. Protected in this way, it can be stored for at least 4 months. In PBS buffered saline with 50% glycerol, the antibodies remain stable for at least 18 months when stored at -80 °C. In addition, the antibody can be dried at 37-50 °C and lyophilised without loss of functionality after re-hydration. It only undergoes denaturation upon prolonged exposure to extreme temperatures, i.e., 70 °C.

The results of the stability analysis of the mAb murine anti-human antibody HER2/ 70.27.58 - i.e., clone 58 are shown in fig. 8.

### B) Stability study of mAb anti-human antibody HER2/70.21.73.67 - clone 67

### • Method of storage of antibodies HER2170.21. 73.67 - clone 67

The analysed antibody was stored long-term in a low-temperature freezer at -80°C after purification. With that said, the antibody was stored in buffer containing 50% (v/v) glycerol, the standard possible use of 30-50% glycerol.

### • Preliminary stability studies of the mAb anti-human HER2/70.21.73.67 antibody

A preliminary "activity" analysis was carried out by testing the HER2 antigen-binding capacity of the mAb anti-human HER2/70.27.58 antibody stored with the addition of different shielding agents and under different temperature conditions. Based on the results of the analyses, it was shown that the mAb anti-human HER2/70.27.58 antibody stored for 18 months in PBS:glycerol buffer at a volume ratio of 1:1 at -80°C showed an antigen-binding capacity similar to that of the sample on day 1 from the date of purification. This allowed the minimum shelf life (expiry date) of this antibody to be set at 18 months from the date of purification and, similarly, for the anti-human mAb antibody HER2/70.21.73.67 initially for an equivalent period, until samples stored for ∼year can be obtained and appropriate analyses performed for this anti-HER2 antibody clone.

### • Comparative study of the stability of the mAb anti-human HER2/70.21.73.67 antibody - specific studies

Verification of antibody stability over time was performed by checking their biological functionality, i.e., ability to bind to the HER2 antigen. Analysis was performed using the direct ELISA technique on antibodies from different lots (purified independently) after approximately one year of storage at -80°C in buffer conditions: PBS+ 50% glycerol. For each lot of antibodies analysed, the parameter ED50 (median effective dose) was determined, representing the amount of antibody (µg) that binds half of the available antigen for a curve in the range 0 - 0.5 µg. Thus, ED50 is a parameter for assessing antibody functionality, and the lower its value, the higher the "activity" of the antibody. The ED50 value from the analysis refers to the range, determined from all results for the antibody lots tested to date. The results are shown in Table 1.

**Table 1. Results of ELISA analysis of different lots of a-HER2/70.21.73.67 antibody and comparison of ED50 parameter values against the acceptable range. The acceptable range was considered to be 0.00150 - 0.00567 for samples 1 - 3 and 0.00156 - 0.00565 for sample 4.**

| **No.** | **Antibody** | **Lot number** | **Storage time at -80°C [days]** | **ED50 value** | **ED50 in set range** |
|---|---|---|---|---|---|
| 1. | mAb a-HER2 70.21.73.67 (BIO-ABH-02) | SDS-20211231 | 352 | 0.0043 | YES |
| 2. | mAb a-HER2 70.21.73.67 (BIO-ABH-02) | SDS-20220126 | 340 | 0.0043 | YES |
| 3. | mAb a-HER2 70.21.73.67 (BIO-ABH-02) | SDS-20220120 | 341 | 0.0040 | YES |
| 4. | mAb a-HER2 70.21.73.67 (BIO-ABH-02) | SDS-20211201 | 430 | 0.0046 | YES |

### • Conclusions

The lots of mAb anti-human HER2/70.21.73.67 antibodies tested have normal antigen binding capacity. Based on the results, long-term storage conditions were confirmed: -80°C and PBS+50% glycerol buffer as optimal for maintaining the "activity" of the antibody for at least 14 months. In parallel, the minimum shelf life of the anti-human mAb antibody HER2/70.21.73.67 was set at 1 year and 2 months (14 months).

### Example 4.

### Method of detecting HER2 in a biological sample

In this exemplary embodiment, the method for detecting HER2 in a biological sample according to the invention was carried out using an immunoenzymatic ELISA assay, wherein the first anti-HER2 antibody is a mAb anti-human-HER2/clone 58 antibody, whose heavy chain variable region consists of the amino acid sequence of SEQ ID No. 13 and whose light chain variable region consists of the amino acid sequence of SEQ ID No. 15, and wherein the second anti-HER2 biotin-bound antibody used is the mAb anti-human-HER2/70.21.73.67, whose heavy chain variable region consists of the amino acid sequence of SEQ ID No. 14 and whose light chain variable region consists of the amino acid sequence of SEQ ID No. 16.

The uniqueness of the sequence, the ability of the two antibody clones (i.e., clone 58 and clone 67) to bind distinct fragments of the same antigen and the high stability and resistance to drying have been used in the immunoassay. The principle of this assay is based, for example, on the use of a 96-well plate and protein elements sequentially bound to said plate to form a so-called sandwich. Such an arrangement allows the recognition and quantification of HER2 antigen in a tested sample, with the possibility to measure multiple samples simultaneously and compare them. The 96-well plate is an example, but not the only possible application of the invention in analyses.

The plate is coated with an array of protein adhesins in combination to allow HER2 binding and signal generation from the conjugated with biosensor, as schematically shown in Fig. 9.

For this purpose, a specific murine antibody directed against the human HER2 antigen mAb a-HER2/clone 58 is applied in carbonate buffer directly to the platelet surface.

In this exemplary embodiment, coating was carried out in 96-well plates dedicated to ELISA assay (Nunc MaxiSorp) in a buffer that facilitates adhesion to the plate (Carbonate Buffer NaHCO₃ pH 9.6), filtered through a syringe filter with Ø0.22 nm pores.

A solution of murine anti-human HER2/clone 58 antibody was prepared at a concentration of 1 µg/mL in freshly prepared carbonate buffer. This buffer allows the antibody to bind and stabilise to the surface of the plastic and subsequently attach subsequent assay components. Subsequently, 100 µl of antibody solution per well was applied to the selected wells according to the planned assays and 100 µl of carbonate buffer without antibody to the control wells. It is recommended to perform each measurement in triplicate.

The plate was secured with a disposable adhesive cover and incubated for 1 h at room temperature (RT) on a laboratory cradle. After incubation, the plate was transferred to 4 °C and incubated without shaking for a further 16 h (O/N, overnight). After removal from the refrigerator, the plate was incubated for 1 h at RT on the laboratory cradle to stabilise the temperature.

At the end of incubation, the solution should be removed from all wells using a multichannel pipette and the plate was dried by inverting it on a paper towel.

The spots on the plate surface that did not bind the adhesin are then blocked with a solution of 5% milk in PBS. For this purpose, 100 µl of blocking solution (i.e., 5% (w/v) milk in PBS) was applied to all wells. The plate was then secured with a self-adhesive disposable lid and incubated for 1 h at RT in a laboratory cradle. At the end of the incubation, the solution was removed from all wells using a multichannel pipette and the plate was then dried by inverting on a paper towel.

Next, the plate was subjected to trehalose fixation, whose function is to protect the bound antibody during drying and to stabilise the system during long-term storage. To achieve this, a 100 µl of 5% (w/v) trehalose in blocking solution, i.e., 5% milk in PBS, is applied to all wells. The plate should then be secured with a self-adhesive disposable lid and incubated for 1 h at RT in laboratory cradle. At the end of the incubation, the solution was removed from all wells using a multichannel pipette and the plate was then dried by inverting on a paper towel. The dried plate was left upside down on a paper towel at RT O/N until completely dry.

The plate dried in this way may be stored at 5 °C±3 for at least 4 months. In order to protect the plate during storage, dried plates should be placed individually in sealed aluminium bags (Pet12/A16/Pe100). A sachet of silica gel should be inserted in each pouch in order to reduce moisture. The aluminium pouch should be sealed tightly with a heat sealer, with as little air inside as possible. Each pouch was appropriately labelled afterwards with the necessary information (date, Ab, type of blocking, trehalose).

Detection of recombinant HER2 antigen or native HER2 derived from human body fluid samples (e.g., blood serum, peritoneal fluid), cell lysate or tissue homogenate is carried out by applying the solution to a plate and incubating for one hour at ambient temperature. During this time, HER2 antigen from the solution undergoes binding to the mAb anti-human HER2 clone 58 antibodies immobilised on the plate surface. Free unbound antigen is removed from the plate wells during washing step and afterwards an anti-HER2 antibody in complex with biotin is added to the HER2 and a-HER2/clone 58 antibody conjugate, wherein in this exemplary embodiment biotin-bound anti-HER2 antibody is mAb anti-human HER2/clone 67 in complex with biotin. It is the unique sequence of the antibodies that allows them to bind simultaneously to the same antigen molecule that enables the formation of a "sandwich" and subsequent binding of biotin to avidin and horseradish peroxidase (HRP).

Such biosensor generates a chemiluminescent signal to quantify the level of HER2 bound in the "sandwich". The successive steps of the immunoenzymatic assay are shown as a flowchart in Fig. 10.

In fig. 10 the dashed line indicates the stage at which the procedure can be stopped and then continued at any time. So far, the useability of the coated plate has been confirmed over a period of 4 months after the first steps have been performed, with no loss of functionality (Fig. 11). The possibility of stopping the procedure with the stability of the immobilised components significantly shortens the sample analysis procedure and makes it possible to distribute the coated plates as an intermediate between laboratories to standardise analyses.

The complex structure of the bound protein components amplifies the signal, thereby increasing the sensitivity of the assay and lowering the sensitivity threshold of the ELISA assay to 0.5 ng/ml. The immuno-enzymatic assay constructed in this way allows the quantification of HER2 within the working range of the standard curve of 15.6 - 100 ng/ml. Determination of higher HER2 concentrations is possible by performing dilutions of the tested samples. The use of dilutions of the tested samples allowed the determination of HER2 antigen concentrations as high as 150 µg/ml (data obtained for murine tumours).

Moreover, the coefficient of intra-test and inter-test variability was analysed on the basis of independent repetitions of the experiment, conducted on different days under the same conditions. The intra-test variability was determined to be <10%, while the inter-test variability was <20%. The LOD (level of detection) was experimentally determined at 0.5 ng/ml based on serial dilutions of 0.09 - 1.0 ng/ml. LOQ (level of quantification) was experimentally determined at 1.56 ng/ml.

Although this exemplary embodiment shows the use of the combination of the antibody according to the invention clone 58 together with clone 67, the use of the test procedure in a system with another, including a commercially available antibody, is not excluded.

Table 2 shows a comparison of the effectiveness of the method according to the invention using an immuno-enzymatic assay with commercially available products:

**Table 2: Comparison of the method according to the invention using an immuno-enzymatic assay with commercially available immuno-enzymatic assays:**

| **Manufacture r** | **Catalogu e no.** | **Sensitivity** | **Range** | **Type of sample** | **Reading time (hours)** | **Other** |
|---|---|---|---|---|---|---|
| Abeam | ab283881 | 4.95 pg/ml | 31.25 - 2000 pg/ml | Milk, serum, cell lysate, cell culture medium, hep plasma, EDTA plasma, Cit plasma | 1:30 | |
| Abeam | ab 100510 | 8 pg/ml | 8.19 - 2000 pg/ml | Cell culture supernatant, plasma, serum | - | |
| Abcam | ab237645 | 11 ng/ml | 11.0-300 ng/ml | Plasma, serum | 2:00 | |
| Bio-Techne/ R&D Systems | DHER20 | 14.8 pg/ml | 78.1 - 5000 pg/ml | Cell culture supernatant, cell lysate, serum, EDTA plasma, Heparin plasma, urine, human milk | 6:00 | A cross-reaction was observed |
| BioVision.co m | E4357 | <188pg/ml | 312 - 20000 pg/ml | Serum, plasma, tissue homogenate, cell culture supernatant, body fluids | - | |
| Bosterbio.com | EKC1270 | >5000 cells | >5000 cells | Cells/human, murine, rat | 6:30 | |
| Bosterbio.com | EK0756 | 10 pg/ml | 62.5 - 4000 pg/ml | Cell culture supernatant, cell lysates, serum, plasma (heparin, EDTA) | 6:30 | |
| Cell Signalling/Path Scan | 7310 | - | - | Cell lysate | 6:00 | Phopho-HER2 |
| Creative-biolabs | TM-HMM221 | 62.5 ng/ml | 62.5 - 500 ng/ml | Serum, plasma | - | |
| Eagle Bioscience | AHR31-K01 | 245 pg/ml | 245 - 500 000 ng/ml | Serum, plasma EDTA | 4:30 | |
| Invitrogen | EHERBB 2 | 8 pg/ml | 8.19 - 2000 pg/ml | Plasma, serum, supernatant | 4:45 | |
| Sigma-Aldrich | RAB0173 | 8 pg/ml | 8.19 - 2000 pg/ml | Cell culture supernatant, plasma, serum, urine | 6:30 | |
| SinoBiologica 1 | KIT10004 | 7 pg/ml | 62.5 - 4000 pg/ml | Serum | - | |
| **Assay according to the invention** | **-** | **500.0** | **1560-100 000 pg/ml** | **Serum, cell lysate, tissue homogenate, peritoneal fluid, recombinant antigen suspended in saline** | **5:30** | **-** |

### Example 5.

### Use of ELISA assay for measurement of the tumour marker HER2 levels in the human serum samples

The method according to the invention can be used to measure antigen levels in any solution, which can be either recombinant antigen suspended in saline or direct samples of body fluids.

In this exemplary embodiment, the method according to the invention using the ELISA assay according to Example 4 and antibodies according to Example 1 was used to analyse human serum samples for the measurement of free ECD of HER2. When a HER2-positive breast tumour is present, HER2 levels are dramatically increased both in and around the tumour cells and the extracellular domain of the receptor is released into the circulation. Elevated HER2 levels may be subject to detection in peripheral blood and provide a diagnostic method.

Samples from clinical patients with breast cancer (n=9) and commercially available human serum (NeoBiotech, PB-Pool-150mL-20211214) without confirmed elevated HER2 levels, serving as control, were used in the example analysis. Serum samples were inserted to the wells of a plate coated with mAb anti-human HER2 clone 58 and analysed according to the procedure in example 4. The results are shown in table 3.

**Table 3. Determination of HER2 antigen levels in serum from clinical patients with breast cancer and commercially available control serum. Average HER2 levels were determined by duplicate analysis and presented as average in ng/ml.**

| **No.** | **Patient** | **Average HER2 level [ng/ml]** |
|---|---|---|
| 1. | ER-/PR-/HER2+ breast cancer | 181.4 |
| 2. | ER-/PR-/HER2+ breast cancer | 71.9 |
| 3. | Breast cancer ER+++/PR++/HER2+ | 183.7 |
| 4. | Breast cancer ER+++/PR++/HER2+ | >210.0 |
| 5. | Breast cancer ER+++/PR++/HER2++ _ FISH -. | 161.4 |
| 6. | Breast cancer ER+++/PR++/HER2+ | 165.3 |
| 7. | Breast cancer ER+++/PR++/HER2+ | 129.8 |
| 8. | Breast cancer ER+++/PR++/HER2+ | 195.3 |
| 9. | Simple endometrial hyperplasia | 83.8 |
| 10. | PB-Pool | 29.5 |

Analysis of the results showed that the immunoenzymatic assay plotted the differential antigen content of breast cancer patients in the range of 71 - 215 ng/ml, with a concomitant level of 29.5 ng/ml (1.9%) in a pooled serum sample from healthy donors, indicating a significantly elevated HER2 content in breast cancer patients, relative to values considered physiological (~15 ng/ml) according to current knowledge of the disease picture.

### Use of ELISA assay to measure HER2 tumour marker levels in xenogeneic murine model tumour samples and serum

Next, the immunoenzymatic assay according to Example 4 with antibodies according to Example 1 was used to measure HER2 antigen levels in tumour tissues from mice with xenogeneic transplantation of human tumour cells derived from ovarian cancer (SK-OV-2; HER2-positive, group A) and breast cancer (MDA-MB-231; HER2-negative, group B). Three mice from each group were analysed. During the sample preparation procedure, tumour tissues were subjected to maceration, i.e., crushing of frozen tissue. In order to release the non-cell-bound form of HER2, the tissue material was subjected to a series of washes using a buffer solution (buffers: PBS/PBST/RIPA). After centrifugation of the insoluble cellular components, the supernatant was then applied to a plate with an immobilised mAb anti-human HER2/clone 58 antibody and analysed according to the test procedure as for serum. The results are shown in table 4.

**Table 4. Determination of HER2 antigen levels in tumours in mice induced with SK-OV-3 and MDA-MB-231 cells, performed by the immunoenzymatic assay of the invention. All experiments were performed in three independent replicates and results are presented in ng/ml as averages per experiment together with the coefficient of variation (%CV).**

| | **Group A (SK-OV-3)** | | | **Groupa B (MDA-MB-231)** | | |
|---|---|---|---|---|---|---|
| **Sample** | **Mouse 1** | **Mouse 2** | **Mouse 3** | **Mouse 1** | **Mouse 2** | **Mouse 3** |
| **Serum** soluble HER2 | 162.1 (4.6%) | 129.5 (6.6%) | 166.1 (7.6%) | <LOD | <LOD | <LOD |
| **Tumour** soluble HER2 | 80825.0 (4.4%) | 50778.1 (3.4%) | 10102.9 (5.7%) | <LOD | <LOD | <LOD |
| **Tumour** Cell-associated HER2 | 101625.4 (4.8%) | 41389.2 (8.6%) | 82186.4 (1.6%) | <LOD | <LOD | <LOD |

The obtained results showed the absence of antigen in mice induced with HER2-negative cells (MDA-MB-231), which is consistent with literature data. At the same time, HER2 levels in mice induced with HER2-positive SK-OV-3 cells were dramatically elevated and ranged from 10 - 80 µg/ml. The values obtained for HER2 levels are within the ranges presented in the literature and are consistent with existing knowledge of tumour development and the molecular biology of the HER2 tyrosine receptor. These measurements confirmed the functionality of the immunoenzymatic ELISA assay according to the invention using antibodies according to the invention in patient material and the potential for use in cancer diagnosis to determine tumour type (HER2-positive or HER2-negative).

### Use of ELISA assay to measure HER2 in culture medium samples and cell lysates of lines MDA-MB-231, SK-OV-3 and SK-BR-3

The immunoenzymatic assay according to Example 4 with antibodies according to Example 1 was then used to measure HER2 levels in samples of culture medium and cell lysates of the MDA-MB-231, SK-OV-3 and SK-BR-3 lines.

The method according to the invention using ELISA assay remains functional for most types of biological materials, including blood serum, tissue homogenates, as well as cell lysates and cell supernatants/supernatants. Analysis performed on material from the MDA-MB-231, SK-OV-3 and SK-BR-3 lines allowed confirmation of the presence of the HER2 antigen and its quantification in culture medium and cell lysates.

Cells were seeded at 3×10⁵ cells/well in a 12-well plate and cultured under standard conditions. At three time points: after 24, 48 and 72 h, both culture medium and cells were harvested and cell lysate in RIPA buffer was prepared from the confluent cells. The resulting culture medium and cell lysate samples were then analysed by ELISA assay.

The HER2 secretion by adherent cells is maintained at low levels, but antigen levels in lysates significantly differentiate these lines. In MDA-MB-231 cells, treated as HER2-negative, antigen levels are maintained at low physiological levels, while in HER2-positive SK-OV-3 and SK-BR-3 cells, the levels are significantly higher. The recorded HER2 receptor level in the lysates is 1000-fold higher than in the culture medium (ng/ml in the medium vs µg/ml in the lysates), which may be related to the significant dilution of HER2 in the culture medium. In addition, the increase in HER2 levels is evident over time, with the highest concentrations observed after 72 h. The results obtained are shown in Table 5.

**Table 5. Determination of HER2 antigen levels in culture medium samples and cell lysates of lines MDA-MB-231, SK-OV-3 and SK-BR-3. Analysis was performed 24, 48 and 72 h after cell seeding. Cells were counted and results presented as HER2 concentration in ng/ml. Lysate samples for ELISA were diluted 1:2000.**

| **Cell line** | **Type of sample** | **Growth time** | **HER2 [ng/ml]** |
|---|---|---|---|
| MDA-MB-231 | culture medium | 24 h | 1.28 |
| | | 48 h | 6.20 |
| | | 72 h | 12.56 |
| | cell lysate | 24 h | 3700.59 |
| | | 48 h | 13783.75 |
| | | 72 h | 14005.23 |
| SK-OV-3 | culture medium | 24 h | 9.79 |
| | | 48 h | 18.97 |
| | | 72 h | 25.68 |
| | lysate | 24 h | 9717.43 |
| | | 48 h | 13827.54 |
| | | 72 h | 21130.25 |
| SK-BR-3 | culture medium | 24 h | 63,22 |
| | | 48 h | 102,38 |
| | | 72 h | 145,75 |
| | cell lysate | 24 h | 17181.01 |
| | | 48 h | 31770.42 |
| | | 72 h | 22896.74 |

The obtained results are in agreement with literature data and the HER2-positive nature of the SK-OV-3 and SK-BR-3 lines, as well as confirming the versatility of the application of the ELISA assay according to the invention using antibodies according to the invention for analysing of various biological materials.

### Example 6.

### Evaluation of the cytotoxicity of antibodies according to the invention on human cells

The mAb anti-human HER2/clone 70.27.58 and anti-human HER2/clone 70.21.73.67 antibodies were analysed in the context of their cytotoxicity and effects on *in vitro* cell culture. In the MTT tetrazolium salt reduction assay, syngeneic cells of the MDA-MB-231 and SK-OV-3 lines were exposed for 24 h to antibodies at increasing concentrations in the range 0.1 ng/ml - 100 µg/ml and their survival was analysed. Living cells efficiently metabolise the tetrazolium salt (3-(4,5-dimethylthiazol-2-yl)-2,5-diphenyltetrazolium bromide) to insoluble formazan through mitochondrial dehydrogenase activity. Detection of the performed MTT test is based on spectrophotometric measurement of the coloured reaction product at 570 nm. The results are shown in fig. 12.

The obtained results were analysed against measurements for untreated cells and presented as % of control. Measurements of RPMI substrate control and cells treated with a native murine antibody were used for comparison.

The MTT assay showed no significant differences between the effects of the tested anti-human HER2 antibodies and IgG class antibodies from non-immunized mice. Cell survival rates for both the MDA-MB-231 and SK-OV-3 lines ranged from ~100% at the tested concentrations of 0.1 ng/ml - 10 µg/ml. Only at the highest antibody concentration (100 µg/ml) was a reduction in cell survival was observed under the influence of the antibodies added to the medium, but this effect was also observed in SK-OV-3 for control murine IgG antibodies. This may suggest a general negative effect of added exogenous factors at high concentrations and not necessarily a cytotoxic effect of the anti-HER2 antibodies analysed.

This analysis showed that the mAb anti-human HER2/clone 70.27.58 and anti-human HER2/clone 70.21.73.67 antibodies do not show cytotoxicity towards MDA-MB-231 and SK-OV-3 cells in the *in vitro* culture. Their ability to bind antigen may therefore have potential applications in new therapies, e.g., as a drug carrier.

### Example 7.

### Use of antibodies according to the invention and F(ab')2 fragments to produce an optoelectronic biosensor

In this exemplary embodiment, mAb anti-human HER2/clone 70.27.58 antibodies or their F(ab')2 fragments were used.

The antibodies according to the invention can be used in the production of an optoelectronic biosensor due to their ability to retain full functionality and ability to bind HER2 in an immobilised form to the modified surface of the optical fibre. Anti-human HER2 antibodies immobilised on the surface of the biosensor, acting as adhesins, capture even small, nanogram quantities of HER2 antigen from solution. Sensitive fibre-optic technology, on the other hand, makes it possible to record signal, which is wavelength changes given in nanometres.

In exemplary analyses performed herein, 4 optoelectronic sensors of known design (fig. 13A), based on fibre-optic technology using the refractive index of the substance to be measured in solution (so-called Reflective Index fibreoptics sensors), were tested. Briefly, these biosensors were coated with an anti-human HER2 antibody according to the invention and, after blocking the surface with a non-specific protein, were tested in three increasing concentrations of HER2. Wavelength changes were recorded and tabulated. Analogous tests were also performed for sensors coated with F(ab')2 fragments of anti-human HER2 antibodies (fig. 13B). For all sensors, wavelength changes were recorded as a result of HER2 binding to the biosensor surface.

HER2 biosensor technology is sensitive enough to detect nanogram (including physiological) amounts of antigen, as well as elevated HER2 levels as a result of pathological conditions, including in HER2-positive breast cancer. Wavelength changes in response to increasing antigen concentration show a linear relationship (fig. 14), both when using sensors coated with full anti-human HER2 antibodies (A) and F(ab')2 fragments (fig. 14B).

Performed analyses confirmed that the sensitivity of the biosensors with anti-HER2 antibodies according to the invention immobilised on their surface as a function of adhesins, combined with the possibility of application in the living organism, resulted in the innovative technology providing a modern tool for oncological diagnosis. In this case, the HER2-detecting biosensor can be used as a less invasive and less time-consuming solution than a biopsy, monitoring in real time, not only the level of the breast cancer marker in the tumour's surroundings, but also drug delivery and efficacy. The biosensor, inserted via a metal guidewire into the tumour area, allows the result to be recorded within ~20 minutes.

### Example 8.

### Therapeutic use of nucleotide sequences encoding antibody variable regions for CAR-T production

The variable regions of the anti-HER2 monoclonal antibodies according to inventions, thanks to their antigen-binding specificity and proven lack of cytotoxicity, can potentially find their application in personalised CAR-T therapy. The nucleic acid molecule encoding the variable region of the anti-HER2 antibody in the integrating vector can be introduced into the T lymphocyte by electroporation methods, using gammaretroviral vectors, lentiviral vectors or based on transposon/transposase systems, ensuring stable integration into the genome of T lymphocytes and their progenitor cells.

Whereby, variable regions of antibodies with a nucleotide sequence selected from SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28 can be used to produce CAR-T.

The HER2 antigen strongly expressed on the surface of tumour cells is recognised by the chimeric CAR-T receptor due to a fragment of the anti-HER2 antibody light chain variable sequence according to the invention, which can potentially lead to activation of the immune response and removal of such cells from the patient's body. Since HER2 is also localised in small amounts in healthy cells, it would be necessary to use iCAR (inhibitory chimeric antigen receptors) technology to protect the patient's body from the effects of an autoimmune and immune system response directed against healthy cells. The iCAR technology uses a typical CAR receptor (a-CAR) directed against a tumour cell surface antigen that may also be present in healthy cells and the iCAR protective system, exploiting the fact that certain antigens are lost in tumour cells due to a loss-of heterozygosity (LOH) chromosomal mechanism. In this case, healthy cells expressing an iCAR antigen (e.g., HLA-A2 or programmed cell death protein 1, PD-1) are protected, while cancer cells lacking this antigen are killed. Such a two-step protection system limits the cytotoxicity of CAR-T against healthy cells and carries enormous potential for use in solid tumours.

### Example 9.

### Therapeutic use of humanised anti-HER2 antibodies according to the invention in antibody-drug conjugates

In ADC conjugates, anti-HER2 antibodies according to the invention undergo covalent binding to the drug molecule via a linker. Examples of anti-cancer drugs used in conjugates include the microtubule inhibitor auristatin A (MMAE), DNA-binding calicheamycin, the topoisomerase 1 inhibitor SN-38 or the glucocorticoid receptor modulator GRM. Their function is to interfere with the normal functioning of cells and, therefore, their action relates to the basic cellular processes. There are many variations of ADCs, depending on the type and form of the antibody recognising the antigen, e.g., diabody, Fab, scFV, bicyclic peptides, as well as the number of drug molecules carried simultaneously or their type, including nanocarriers, liposomes and polymeric molecules. ADC therapy is popular, not least because of the widespread use of its components, i.e., chemotherapeutics and monoclonal antibodies in therapy. Conjugates therefore remain a slightly improved, more selective and more effective form of a well-known and long-standing cancer therapy.

Anti-HER2 antibodies according to invention could potentially be used to prepare ADCs with therapeutic potential. In order to increase the efficiency of transport in the human body, while reducing the risk of removal by the patient's immune system, the anti-HER2 antibodies should be humanised. In such a process, an antibody hybrid is created in which the human antibody forms the base, while the variable regions - which specifically recognise the antigen - are derived from an antibody of a different species. The use of the sequence of hypervariable regions of anti-HER2 antibodies according to invention in humanised bio-drug molecules, will enable the preservation of the specificity of HER2 binding, comparable to tested antibodies (murine antibodies), and the risk of recognition by the patient's immune system as a foreign molecule, leading to its removal, is reduced. The innovation of such use of the unique variable regions of the presented antibodies is based on their specificity towards HER2 and the possibility of selecting the appropriate type of conjugated cytotoxin.

The monoclonal anti-human HER2/clone 70.27.58 and anti-human HER2/clone 70.21.73.67 antibodies are distinguished by their specificity towards HER2 in tandem, binding simultaneously to distinct epitopes of the same antigen. Their functionality and high biological stability, as well as their ability to recognise both native and denatured antigen, enables a number of their applications in the fields of molecular and cell biology and modern diagnostic and therapeutic technologies. The anti-human HER2/clone 70.27.58 and anti-human HER2/clone 70.21.73.67 antibodies remain functional both in solution and in immobilised form, which also leaves enormous potential for use in techniques not presented in this description of the invention.

### List of sequences

Sequence number (SEQ ID No.): 1
   Type: amino acid sequence
   Length: 8
   Description: CDR1 of the heavy chain variable region of the antibody of the invention according to the first embodiment
   Sequence: GYSFTGYN
Sequence number (SEQ ID No.): 2
   Type: amino acid sequence
   Length: 8
   Description: CDR1 of the heavy chain variable region of the antibody of the invention according to the second embodiment
   Sequence: GYSFTDYN
Sequence number (SEQ ID No.): 3
   Type: amino acid sequence
   Length: 7
   Description: CDR2 of the heavy chain variable region of the antibody of the invention according to the first embodiment
   Sequence: IDPNYGT
Sequence no. (SEQ ID No.): 4
   Type: amino acid sequence
   Length: 8
   Description: CDR2 of the heavy chain variable region of the antibody of the invention according to the second embodiment
   Sequence: IDHYNGNT
Sequence number (SEQ ID No.): 5
   Type: amino acid sequence
   Length: 13
   Description: CDR3 of the heavy chain variable region of the antibody of the invention according to the first embodiment
   Sequence: ARRKVNYEAWFAF
Sequence no. (SEQ ID No.): 6
   Type: amino acid sequence
   Length: 11
   Description: CDR3 of the heavy chain variable region of the antibody of the invention according to the second embodiment
   Sequence: ARHYDYGAMDY
Sequence number (SEQ ID No.): 7
   Type: amino acid sequence
   Length: 10
   Description: CDR1 of the light chain variable region of the antibody of the invention according to the first embodiment
   Sequence: ESVDSYGNSF
Sequence no. (SEQ ID No.): 8
   Type: amino acid sequence
   Length: 6
   Description: CDR1 of the light chain variable region of the antibody of the invention according to the second embodiment
   Sequence: QNVGTN
Sequence no. (SEQ ID No.): 9
   Type: amino acid sequence
   Length: 3
   Description: CDR2 of the light chain variable region of the antibody of the invention according to the first embodiment
   Sequence: RAS
Sequence number (SEQ ID No.): 10
   Type: amino acid sequence
   Length: 3
   Description: CDR2 of the light chain variable region of the antibody of the invention according to the second embodiment
   Sequence: SAS
Sequence no. (SEQ ID No.): 11
   Type: amino acid sequence
   Length: 9
   Description: CDR3 of the light chain variable region of the antibody of the invention according to the first embodiment
   Sequence: QQSNEDPFT
Sequence no. (SEQ ID No.): 12
   Type: amino acid sequence
   Length: 9
   Description: CDR3 of the light chain variable region of the antibody of the invention according to the second embodiment
   Sequence: QHYYIYPLT
Sequence no. (SEQ ID No.): 13
   Type: amino acid sequence
   Length: 119
   Description: the heavy chain variable region of the antibody of the invention according to the first embodiment
   Sequence: 
EVQLQQSGPELEKPGASVKISCKASGYSFTGYNMNWVKQSNGKSLEWIGTIDPNYGTSYNQK
FKGKATLTVDKSSSTAYMHLKSLTSEDSSVYYCARRKVNYEAWFAFWGQGTLVTVSA
Sequence no. (SEQ ID No.): 14
   Type: amino acid sequence
   Length: 118
   Description: the heavy chain variable region of the antibody of the invention according to the second embodiment
   Sequence: 
EIQLQQSGPELVKPGASVRVSCKASGYSFTDYNMYWVRQSQGESLEWIGYIDHYNGNTGYN
QKFRDKATLTVDKASSTAFMHLNSLTSEDSAVYYCARHYDYGAMDYWGQGTSVTVSS
Sequence number (SEQ ID No.): 15
   Type: amino acid sequence
   Length: 112
   Description: the light chain variable region of the antibody of the invention according to the first embodiment
   Sequence: 
DIVLTQSPASLAVSLGQRATISCRASESVDSYGNSFMHWYQQKPGQPPKLLIYRASNLESGIPA
RFSGSGSRTDFTLTINPVEADDVATYYCQQSNEDPFTFGSGTKLEIKR
Sequence number (SEQ ID No.): 16
   Type: amino acid sequence
   Length: 108
   Description: the light chain variable region of the antibody of the invention according to the second embodiment
   Sequence: 
DIVMTQSQKFMSTSVGDRVSVTCKASQNVGTNVAWYQQKPGQSPKALIYSASYRYSGVPDR
FTGSGSGTDFTLTISTVQSEDLAEYFCQHYYIYPLTFGAGTKLELKR
Sequence no. (SEQ ID No.): 17
   Type: nucleotide sequence
   Length: 24
   Description: CDR1 of the heavy chain variable region of the antibody of the invention according to the first embodiment
   Sequence: GGTTACTCATTCACTGGCTACAAC
Sequence No. (SEQ ID No.): 18
   Type: nucleotide sequence
   Length: 24
   Description: CDR1 of the heavy chain variable region of the antibody of the invention according to the second embodiment
   Sequence: GGTTACTCATTCACTGACTACAAC
Sequence no. (SEQ ID No.): 19
   Type: nucleotide sequence
   Length: 24
   Description: CDR2 of the heavy chain variable region of the antibody of the invention according to the first embodiment
   Sequence: ATTGATCCTAACTATGGTGGTACT
Sequence No. (SEQ ID No.): 20
   Type: nucleotide sequence
   Length: 24
   Description: CDR2 of the heavy chain variable region of the antibody of the invention according to the second embodiment
   Sequence: ATTGATCATTACAATGGAAATACT
Sequence No. (SEQ ID No.): 21
   Type: nucleotide sequence
   Length: 39
   Description: CDR3 of the heavy chain variable region of the antibody of the invention according to the first embodiment
   Sequence: GCAAGACGGAAGGTTAACTACGAGGCCTGGTTTGCTTTC
Sequence No. (SEQ ID No.): 22
   Type: nucleotide sequence
   Length: 33
   Description: CDR3 of the heavy chain variable region of the antibody of the invention according to the second embodiment
   Sequence: GCAAGACACTACGACTACGGTGCTATGGACTAC
Sequence No. (SEQ ID No.): 23
   Type: nucleotide sequence
   Length: 30
   Description: CDR1 of the light chain variable region of the antibody of the invention according to the first embodiment
   Sequence: GAAAGTGTTGATAGTTATGGCAATAGTTTT
Sequence No. (SEQ ID No.): 24
   Type: nucleotide sequence
   Length: 18
   Description: CDR1 of the light chain variable region of the antibody of the invention according to the second embodiment
   Sequence: CAGAATGTGGGTACTAAT
Sequence No. (SEQ ID No.): 25
   Type: nucleotide sequence
   Length: 9
   Description: CDR2 of the light chain variable region of the antibody of the invention according to the first embodiment
   Sequence: CGTGCATCC
Sequence No. (SEQ ID No.): 26
   Type: nucleotide sequence
   Length: 9
   Description: CDR2 of the light chain variable region of the antibody of the invention according to the second embodiment
   Sequence: TCGGCATCC
Sequence No. (SEQ ID No.): 27
   Type: nucleotide sequence
   Length: 27
   Description: CDR3 of the light chain variable region of the antibody of the invention according to the first embodiment
   Sequence: CAGCAAAGTAATGAGGATCCATTCACG
Sequence No. (SEQ ID No.): 28
   Type: nucleotide sequence
   Length: 27
   Description: CDR3 of the light chain variable region of the antibody of the invention according to the second embodiment
   Sequence: CAGCATTATTACATCTATCCGCTCACG
Sequence number (SEQ ID No.): 29
   Type: amino acid
   Length: 474
   Description: The heavy chain of the antibody of the invention according to the first embodiment Sequence: 
MGWTWIFILILSVTTGVHSEVQLQQSGPELEKPGASVKISCKASGYSFTGYNMNWVKQSNGK
SLEWIGTIDPNYGTSYNQKFKGKATLTVDKSSSTAYMHLKSLTSEDSSVYYCARRKVNYEAW
FAFWGQGTLVTVSAAKTTPPSVYPLAPGCGDTTGSSVTLGCLVKGYFPESVTVTWNSGSLSSS
VHTFPALLQSGLYTMSSSVTVPSSTWPSQTVTCSVAHPASSTTVDKKLEPSGPISTINPCPPCKE
CHKCPAPNLEGGPSVFIFPPNIKDVLMISLTPKVTCVVVDVSEDDPDVQISWFVNNVEVHTAQ
TQTHREDYNSTIRVVSTLPIQHQDWMSGKEFKCKVNNKDLPSPIERTISKIKGLVRAPQVYILP
PPAEQLSRKDVSLTCLVVGFNPGDISVEWTSNGHTEENYKDTAPVLDSDGSYFIYSKLNMKTS
KWEKTDSFSCNVRHEGLKNYYLKKTISRTPGK
Sequence number (SEQ ID No.): 30
   Type: amino acid
   Length: 473
   Description: The heavy chain of the antibody of the invention according to the second embodiment Sequence: 
MEWSWIFLFLLSGTTGVHSEIQLQQSGPELVKPGASVRVSCKASGYSFTDYNMYWVRQSQGE
SLEWIGYIDHYNGNTGYNQKFRDKATLTVDKASSTAFMHLNSLTSEDSAVYYCARHYDYGA
MDYWGQGTSVTVSSAKTTPPSVYPLAPGCGDTTGSSVTLGCLVKGYFPESVTVTWNSGSLSS
SVHTFPALLQSGLYTMSSSVTVPSSTWPSQTVTCSVAHPASSTTVDKKLEPSGPISTINPCPPCK
ECHKCPAPNLEGGPSVFIFPPNIKDVLMISLTPKVTCVVVDVSEDDPDVQISWFVNNVEVHTA
QTQTHREDYNSTIRVVSTLPIQHQDWMSGKEFKCKVNNKDLPSPIERTISKIKGLVRAPQVYIL
PPPAEQLSRKDVSLTCLVVGFNPGDISVEWTSNGHTEENYKDTAPVLDSDGSYFIYSKLNMKT
SKWEKTDSFSCNVRHEGLKNYYLKKTISRTPGK
Sequence number (SEQ ID No.): 31
   Type: amino acid
   Length: 238
   Description: The light chain of the antibody of the invention according to the first embodiment Sequence: 
METDTLLLWVLLLWVPGSTGDIVLTQSPASLAVSLGQRATISCRASESVDSYGNSFMHWYQQ
KPGQPPKLLIYRASNLESGIPARFSGSGSRTDFTLTINPVEADDVATYYCQQSNEDPFTFGSGT
KLEIKRADAAPTVSIFPPSSEQLTSGGASVVCFLNNFYPKDINVKWKIDGSERQNGVLNSWTD
QDSKDSTYSMSSTLTLTKDEYERHNSFTCEATHKTSTSPIVKSFNRNEC
Sequence number (SEQ ID No.): 32
   Type: amino acid
   Length: 234
   Description: The light chain of the antibody of the invention according to the second embodiment
   Sequence: 
MESQTQVFVYMLLWLSGVDGDIVMTQSQKFMSTSVGDRVSVTCKASQNVGTNVAWYQQK
PGQSPKALIYSASYRYSGVPDRFTGSGSGTDFTLTISTVQSEDLAEYFCQHYYIYPLTFGAGTK
LELKRADAAPTVSIFPPSSEQLTSGGASVVCFLNNFYPKDINVKWKIDGSERQNGVLNSWTDQ
DSKDSTYSMSSTLTLTKDEYERHNSYTCEATHKTSTSPIVKSFNRNEC
Sequence number (SEQ ID No.): 33
   Type: nucleotide
   Length: 1532
   Description: The heavy chain of the antibody of the invention according to the first embodiment Sequence: 
ATGGGATGGACCTGGATCTTTATTTTAATCCTGTCAGTAACTACAGGTGTCCACTCTGAGG
TCCAGCTGCAGCAGTCTGGACCTGAGCTGGAGAAGCCTGGCGCTTCAGTGAAGATATCCT
GCAAGGCTTCTGGTTACTCATTCACTGGCTACAACATGAACTGGGTGAAGCAGAGCAATG
GAAAGAGTCTTGAGTGGATTGGAACTATTGATCCTAACTATGGTGGTACTCTTCTGGTTAC
TCATTCACTGGCTACAACATGAACTGGGTGAAGCAGAGCAATGGAAAGAGTCTTGAGTG
GATTGGAACTATTGATCCTAACTATGGTGGTACTAGCTACAACCAGAAGTTCAAGGGCAA
GGCCACATTGACTGTAGACAAATCCTCCAGCACAGCCTACATGCACCTCAAGAGCCTGAC
ATCTGAGGACTCTTCAGTCTATTACTGTGCAAGACGGAAGGTTAACTACGAGGCCTGGTT
TGCTTTCTGGGGCCAAGGGACTCTGGTCACTGTCTCTGCAGCCAAAACAACACCCCCATC
AGTCTATCCACTGGCCCCTGGGTGTGGAGATACAACTGGTTCCTCCGTGACTCTGGGATG
CCTGGTCAAGGGCTACTTCCCTGAGTCAGTGACTGTGACTTGGAACTCTGGATCCCTGTCC
AGCAGTGTGCACACCTTCCCAGCTCTCCTGCAGTCTGGACTCTACACTATGAGCAGCTCA
GTGACTGTCCCCTCCAGCACCTGGCCAAGTCAGACCGTCACCTGCAGCGTTGCTCACCCA
GCCAGCAGCACCACGGTGGACAAAAAACTTGAGCCCAGCGGGCCCATTTCAACAATCAA
CCCCTGTCCTCCATGCAAGGAGTGTCACAAATGCCCAGCTCCTAACCTCGAGGGTGGACC
ATCCGTCTTCATCTTCCCTCCAAATATCAAGGATGTACTCATGATCTCCCTGACACCCAAG
GTCACGTGTGTGGTGGTGGATGTGAGCGAGGATGACCCAGACGTCCAGATCAGCTGGTTT
GTGAACAACGTGGAAGTACACACAGCTCAGACACAAACCCATAGAGAGGATTACAACAG
TACTATCCGGGTGGTCAGCACCCTCCCCATCCAGCACCAGGACTGGATGAGTGGCAAGGA
GTTCAAATGCAAGGTCAACAACAAAGACCTCCCATCACCCATCGAGAGAACCATCTCAA
AAATTAAAGGGCTAGTCAGAGCTCCACAAGTATACATCTTGCCGCCACCAGCAGAGCAGT
TGTCCAGGAAAGATGTCAGTCTCACTTGCCTGGTCGTGGGCTTCAACCCTGGAGACATCA
GTGTGGAGTGGACCAGCAATGGGCATACAGAGGAGAACTACAAGGACACCGCACCAGTC
CTGGACTCTGACGGTTCTTACTTCATATATAGCAAGCTCAATATGAAAACAAGCAAGTGG
GAGAAAACAGATTCCTTCTCATGCAACGTGAGACACGAGGGTCTAAAAAATTACTACCTG
AAGAAGACCATCTCCCGGACTCCGGGTAAATGA
Sequence number (SEQ ID No.): 34
   Type: nucleotide
   Length: 1452
   Description: The heavy chain of the antibody of the invention according to the second embodiment Sequence: 
ATGGAATGGAGCTGGATCTTTCTCTTCCTCCTGTCAGGAACTACAGGTGTCCACTCTGAGA
TCCAGCTGCAGCAGTCTGGACCTGAGCTGGTGAAGCCTGGGGCTTCAGTGAGGGTATCCT
GCAAGGCTTCTGGTTACTCATTCACTGACTACAACATGTACTGGGTGAGGCAGAGCCAAG
GAGAGAGCCTTGAGTGGATTGGATATATTGATCATTACAATGGAAATACTGGCTATAACC
AGAAGTTCAGGGACAAGGCCACATTGACTGTTGACAAGGCCTCCAGCACAGCCTTCATGC
 
ATCTCAACAGCCTGACATCTGAGGACTCTGCAGTCTATTACTGTGCAAGACACTACGACT
ACGGTGCTATGGACTACTGGGGTCAAGGAACCTCAGTCACCGTCTCCTCAGGTCAAGGAA
CCTCAGTCACCGTCTCCTCAGCCAAAACAACACCCCCATCAGTCTATCCACTGGCCCCTG
GGTGTGGAGATACAACTGGTTCCTCCGTGACTCTGGGATGCCTGGTCAAGGGCTACTTCC
CTGAGTCAGTGACTGTGACTTGGAACTCTGGATCCCTGTCCAGCAGTGTGCACACCTTCCC
AGCTCTCCTGCAGTCTGGACTCTACACTATGAGCAGCTCAGTGACTGTCCCCTCCAGCACC
TGGCCAAGTCAGACCGTCACCTGCAGCGTTGCTCACCCAGCCAGCAGCACCACGGTGGAC
AAAAAACTTGAGCCCAGCGGGCCCATTTCAACAATCAACCCCTGTCCTCCATGCAAGGAG
TGTCACAAATGCCCAGCTCCTAACCTCGAGGGTGGACCATCCGTCTTCATCTTCCCTCCAA
ATATCAAGGATGTACTCATGATCTCCCTGACACCCAAAGTCACGTGTGTGGTGGTGGATG
TGAGCGAGGATGACCCAGACGTCCAGATCAGCTGGTTTGTGAACAACGTGGAAGTACAC
ACAGCTCAGACACAAACCCATAGAGAGGATTACAACAGTACTATCCGGGTGGTCAGCAC
CCTCCCCATCCAGCACCAGGACTGGATGAGTGGCAAGGAGTTCAAATGCAAGGTCAACA
ACAAAGACCTCCCATCACCCATCGAGAGAACCATCTCAAAAATTAAAGGGCTAGTCAGA
GCTCCACAAGTATACATCTTGCCGCCACCAGCAGAGCAGTTGTCCAGGAAAGATGTCAGT
CTCACTTGCCTGGTCGTGGGCTTCAACCCTGGAGACATCAGTGTGGAGTGGACCAGCAAT
GGGCATACAGAGGAGAACTACAAGGACACCGCACCAGTCCTGGACTCTGACGGTTCTTA
CTTCATATATAGCAAGCTCAATATGAAAACAAGCAAGTGGGAGAAAACAGATTCCTTCTC
ATGCAACGTGAGACACGAGGGTCTGAAAAATTACTACCTGAAGAAGACCATCTCCCGGA
CTCCGGGTAAATGA
Sequence number (SEQ ID No.): 35
   Type: nucleotide
   Length: 717
   Description: The light chain of the antibody of the invention according to the first embodiment Sequence: 
ATGGAGACAGACACACTCCTGCTATGGGTGCTGCTGCTCTGGGTTCCAGGTTCCACAGGT
GACATTGTGCTGACCCAATCTCCAGCTTCTTTGGCTGTGTCTCTAGGGCAGAGGGCCACC
ATATCCTGCAGAGCCAGTGAAAGTGTTGATAGTTATGGCAATAGTTTTATGCATTGGTAC
CAGCAGAAACCAGGACAGCCACCCAAACTCCTCATCTATCGTGCATCCAACCTAGAATCT
GGGATCCCTGCCAGGTTCAGTGGCAGTGGGTCTAGGACAGACTTCACCCTCACCATTAAT
CCTGTGGAGGCTGATGATGTTGCAACCTATTACTGTCAGCAAAGTAATGAGGATCCATTC
ACGTTCGGCTCGGGGACAAAGTTGGAAATAAAACGGGCTGATGCTGCACCAACTGTATCC
ATCTTCCCACCATCCAGTGAGCAGTTAACATCTGGAGGTGCCTCAGTCGTGTGCTTCTTGA
ACAACTTCTACCCCAAAGACATCAATGTCAAGTGGAAGATTGATGGCAGTGAACGACAA
AATGGCGTCCTGAACAGTTGGACTGATCAGGACAGCAAAGACAGCACCTACAGCATGAG
CAGCACCCTCACGTTGACCAAGGACGAGTATGAACGACATAACAGCTTTACCTGTGAGGC
CACTCACAAGACATCAACTTCACCCATTGTCAAGAGCTTCAACAGGAATGAGTGTTAG
Sequence number (SEQ ID No.): 36
   Type: nucleotide
   Length: 705
   Description: The light chain of the antibody of the invention according to the second embodiment Sequence: 
ATGGAGTCACAGACTCAGGTCTTTGTATACATGTTGCTGTGGTTGTCTGGTGTTGATGGAG
ACATTGTGATGACCCAGTCTCAAAAATTCATGTCCACATCAGTAGGAGACAGGGTCAGCG
TCACCTGCAAGGCCAGTCAGAATGTGGGTACTAATGTAGCCTGGTATCAACAGAAACCAG
GGCAATCTCCTAAAGCACTGATTTATTCGGCATCCTACCGGTACAGTGGAGTCCCTGATC
 
GCTTCACAGGCAGTGGATCTGGGACAGATTTCACTCTCACCATCAGCACTGTGCAGTCTG
AAGACTTGGCAGAGTATTTCTGTCAGCATTATTACATCTATCCGCTCACGTTCGGTGCTGG
GACCAAGCTGGAGCTGAAACGGGCTGATGCTGCACCAACTGTATCCATCTTCCCACCATC
CAGTGAGCAGTTAACATCTGGAGGTGCCTCAGTCGTGTGCTTCTTGAACAACTTCTACCCC
AAAGACATCAATGTCAAGTGGAAGATTGATGGCAGTGAACGACAAAATGGCGTCCTGAA
CAGTTGGACTGATCAGGACAGCAAAGACAGCACCTACAGCATGAGCAGCACCCTCACGT
TGACCAAGGACGAGTATGAACGACATAACAGCTATACCTGTGAGGCCACTCACAAGACA
TCAACTTCACCCATTGTCAAGAGCTTCAACAGGAATGAGTGTTAG
Sequence No. (SEQ ID No.): 37
   Type: nucleotide sequence
   Length: 464
   Description: The variable region of the heavy chain of the antibody of the invention according to the first embodiment
   Sequence: 
GAGGTCCAGCTGCAGCAGTCTGGACCTGAGCTGGAGAAGCCTGGCGCTTCAGTGAAGAT
ATCCTGCAAGGCTTCTGGTTACTCATTCACTGGCTACAACATGAACTGGGTGAAGCAGAG
CAATGGAAAGAGTCTTGAGTGGATTGGAACTATTGATCCTAACTATGGTGGTACTCTTCT
GGTTACTCATTCACTGGCTACAACATGAACTGGGTGAAGCAGAGCAATGGAAAGAGTCTT
GAGTGGATTGGAACTATTGATCCTAACTATGGTGGTACTAGCTACAACCAGAAGTTCAAG
GGCAAGGCCACATTGACTGTAGACAAATCCTCCAGCACAGCCTACATGCACCTCAAGAGC
CTGACATCTGAGGACTCTTCAGTCTATTACTGTGCAAGACGGAAGGTTAACTACGAGGCC
TGGTTTGCTTTCTGGGGCCAAGGGACTCTGGTCACTGTCTCTGCA
Sequence No. (SEQ ID No.): 38
   Type: nucleotide sequence
   Length: 354
   Description: The variable region of the heavy chain of the antibody of the invention according to the second embodiment
   Sequence: 
GAGATCCAGCTGCAGCAGTCTGGACCTGAGCTGGTGAAGCCTGGGGCTTCAGTGAGGGT
ATCCTGCAAGGCTTCTGGTTACTCATTCACTGACTACAACATGTACTGGGTGAGGCAGAG
CCAAGGAGAGAGCCTTGAGTGGATTGGATATATTGATCATTACAATGGAAATACTGGCTA
TAACCAGAAGTTCAGGGACAAGGCCACATTGACTGTTGACAAGGCCTCCAGCACAGCCTT
CATGCATCTCAACAGCCTGACATCTGAGGACTCTGCAGTCTATTACTGTGCAAGACACTA
CGACTACGGTGCTATGGACTACTGGGGTCAAGGAACCTCAGTCACCGTCTCCTCA
Sequence No. (SEQ ID No.): 39
   Type: nucleotide sequence
   Length: 336
   Description: The variable region of the light chain of the antibody of the invention according to the first embodiment Sequence: 
GACATTGTGCTGACCCAATCTCCAGCTTCTTTGGCTGTGTCTCTAGGGCAGAGGGCCACC
ATATCCTGCAGAGCCAGTGAAAGTGTTGATAGTTATGGCAATAGTTTTATGCATTGGTAC
CAGCAGAAACCAGGACAGCCACCCAAACTCCTCATCTATCGTGCATCCAACCTAGAATCT
GGGATCCCTGCCAGGTTCAGTGGCAGTGGGTCTAGGACAGACTTCACCCTCACCATTAAT
CCTGTGGAGGCTGATGATGTTGCAACCTATTACTGTCAGCAAAGTAATGAGGATCCATTC
ACGTTCGGCTCGGGGACAAAGTTGGAAATAAAACGG
Sequence No. (SEQ ID No.): 40
   Type: nucleotide sequence
   Length: 324
   Description: The variable region of the light chain of the antibody of the invention according to the second embodiment
   Sequence:

GACATTGTGATGACCCAGTCTCAAAAATTCATGTCCACATCAGTAGGAGACAGGGTCAGC
GTCACCTGCAAGGCCAGTCAGAATGTGGGTACTAATGTAGCCTGGTATCAACAGAAACCA
GGGCAATCTCCTAAAGCACTGATTTATTCGGCATCCTACCGGTACAGTGGAGTCCCTGAT
CGCTTCACAGGCAGTGGATCTGGGACAGATTTCACTCTCACCATCAGCACTGTGCAGTCT
GAAGACTTGGCAGAGTATTTCTGTCAGCATTATTACATCTATCCGCTCACGTTCGGTGCTG
GGACCAAGCTGGAGCTGAAACGG

## Claims

1. An anti-HER2 antibody, **characterised in that** it comprises:
a) a heavy chain variable region comprising CDR1, CDR2 and CDR3, in which
CDR1 is an amino acid sequence of GYSFTGYN (SEQ ID No. 1) or GYSFTDYN (SEQ ID No. 2),
CDR2 is an amino acid sequence of IDPNYGT (SEQ ID No. 3) or IDHYNGNT (SEQ ID No. 4),
CDR3 is an amino acid sequence of ARRKVNYEAWFAF (SEQ ID No. 5) or ARHYDYGAMDY (SEQ ID No. 6);
b) a light chain variable region comprising CDR1, CDR2 and CDR3 in which
CDR1 is an amino acid sequence of ESVDSYGNSF (SEQ ID No. 7) or QNVGTN (SEQ ID No. 8),
CDR2 is an amino acid sequence of RAS (SEQ ID No. 9) or SAS (SEQ ID No. 10), CDR3 constitutes an amino acid sequence of QQSNEDPFT (SEQ ID No. 11) or QHYYIYPLT (SEQ ID No. 12).

2. An anti-HER2 antibody, **characterised in that** it comprises:
a) a heavy chain with a heavy chain variable region comprising or consisting of an amino acid sequence of SEQ ID No. 13 with 0, 1, 2, 3, 4 or 5 amino acid insertions, deletions, substitutions or additions, or an amino acid sequence of SEQ ID No. 14 with 0, 1, 2, 3, 4 or 5 amino acid insertions, deletions or additions;
b) a light chain with a light chain variable region comprising or consisting of an amino acid sequence of SEQ ID 15 with 0, 1, 2, 3, 4 or 5 amino acid insertions, deletions, substitutions or additions, or an amino acid sequence of SEQ ID 16 with 0, 1, 2, 3, 4 or 5 amino acid insertions, deletions or additions.

3. The antibody according to claim 1 or 2, **characterised in that** its heavy chain variable region consists of the amino acid sequence of SEQ ID No. 13 and its light chain variable region consists of the amino acid sequence of SEQ ID No. 15.

4. The antibody according to claim. 1 or 2, **characterised in that** its heavy chain variable region consists of the amino acid sequence of SEQ ID No. 14 and its light chain variable region consists of the amino acid sequence of SEQ ID No. 16.

5. A nucleic acid molecule encoding the variable region of an antibody according to any of the preceding claims 1 to 4 or a fragment thereof.

6. A method of detecting HER2 in a biological sample, comprising contacting the sample with an HER2-binding protein and detecting a complex, wherein the detection of said complex indicates HER2 expression in the sample, **characterised in that** HER2-binding protein is the antibody according to any of claims 1 to 4.

7. The method according to claim. 6, **characterized in that** contacting of the sample with the HER2-binding protein and the detection of the complex is carried out using an immunoenzymatic ELISA assay, wherein a first anti-HER2 antibody comprises a heavy chain variable region comprising CDR1 with amino acid sequence of SEQ ID No. 1, CDR2 with amino acid sequence of SEQ ID No. 3, CDR3 with amino acid sequence of SEQ ID No. 5, and comprises a light chain variable region comprising CDR1 with amino acid sequence of SEQ ID No. 7, CDR2 with amino acid sequence of SEQ ID No. 9 and CDR3 with amino acid sequence of SEQ ID No. 11, while a second anti-HER2 antibody is a biotin-bound antibody that comprises a heavy chain variable region comprising CDR1 with amino acid sequence of SEQ ID No. 2, CDR2 with amino acid sequence of SEQ ID No. 4 and CDR3 with amino acid sequence of SEQ ID No. 6, and comprises a light chain variable region comprising CDR1 with amino acid sequence of SEQ ID No. 8, CDR2 with amino acid sequence of SEQ ID No. 10 and CDR3 with amino acid sequence of SEQ ID No. 12.

8. The method according to claim 6, **characterized in that** contacting the sample with the HER2-binding protein and detecting the complex is carried out using an immunoenzymatic ELISA, wherein the first anti-HER2 antibody comprises a heavy chain variable region comprising or consisting of amino acid sequence of SEQ ID No. 13, and comprises a light chain variable region comprising or consisting of amino acid sequence of SEQ ID No. 15, and the second anti-HER2 antibody is a biotin-bound antibody that comprises a heavy chain variable region comprising or consisting of amino acid sequence of SEQ ID No. 14 and comprises a light chain variable region comprising or consisting of amino acid sequence of SEQ ID No. 16.

9. The method according to claim 6, **characterised in that** contacting of the sample with the HER2-binding protein and the detection of the complex is carried out using an optoelectronic biosensor with anti-HER2 antibodies according to any of the preceding claims 1 to 4 and/or fragments thereof immobilized on the surface of said sensor.

10. The method according to any of the preceding claims 6 to 9, **characterised in that** biological sample is blood serum, peritoneal fluid, cell lysate, tissue homogenate, cell filtrate, supernatant, or recombinant antigen suspended in solution.

11. An immuno-enzymatic ELISA assay for the detection of HER2 antigen, **characterised in that** at least one antibody according to any of the preceding claims 1 to 4 is used for the detection of antigen.

12. The assay according to claim 11, **characterised in that** two antibodies according to any of the preceding claims 1 to 4 are used for detecting the HER2 antigen, the first of which is an immobilised antibody that comprises a heavy chain variable region comprising CDR1 with amino acid sequence of SEQ ID No. 1, CDR2 with amino acid sequence of SEQ ID No. 3, CDR3 with amino acid sequence of SEQ ID No. 5, and comprises a light chain variable region comprising CDR1 with amino acid sequence of SEQ ID No. 7, CDR2 with amino acid sequence of SEQ ID No. 9 and CDR3 with amino acid sequence of SEQ ID No. 11, while second anti-HER2 antibody is a biotin-bound antibody that comprises a heavy chain variable region contains CDR1 with amino acid sequence of SEQ ID No. 2, CDR2 with amino acid sequence of SEQ ID No. 4 and CDR3 with amino acid sequence of SEQ ID No. 6, and comprises a light chain variable region comprising CDR1 with amino acid sequence of SEQ ID No. 8, CDR2 with amino acid sequence of SEQ ID No. 10 and CDR3 with amino acid sequence of SEQ ID No. 12.

13. An use of antibody according to any of the preceding claims 1 to 4 for producing antibody-drug conjugates.

14. An use of SEQ ID No. 17-28 nucleotide sequences encoding variable regions of the antibodies according to any of the preceding claims 1 to 4 for the CAR-T production.
